(19) **Europäisches Patentamt European Patent Office Office européen des brevets**

(11) **EP 4 729 049 A1**

(12) **EUROPEAN PATENT APPLICATION**

(43) Date of publication:
22.04.2026 Bulletin 2026/17

(21) Application number: 24206603.3

(22) Date of filing: 15.10.2024

(51) International Patent Classification (IPC):
*A61K 9/16* (2006.01)        *A61K 9/20* (2006.01)
*A61K 47/50* (2017.01)

(52) Cooperative Patent Classification (CPC):
(C-Sets available)
**A61K 9/1676; A61K 9/1652; A61K 9/1682;
A61K 9/2054; A61K 47/6951; C08B 37/0015;
C08L 5/16** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Emoviar PharmaCon GmbH
79664 Wehr (DE)**

• **Scheer, Mathias Josef
79664 Wehr (DE)**

(72) Inventor: **Scheer, Mathias Josef
79664 Wehr (DE)**

(74) Representative: **CH Kilger Anwaltspartnerschaft
mbB
Fasanenstraße 29
10719 Berlin (DE)**

(54) **SUSTAINED RELEASE DOSAGE FORM FOR LOW SOLUBILITY COMPOUND AXITINIB AND METHODS FOR PREPARING OF THIS SUSTAINED RELEASE DOSAGE FORM**

(57)    The present invention relates to a solid oral pharmaceutical for Axitinib (API) or any other Kinase Inhibitor API with a novel, well defined method to enhance solubility of active pharmaceutical ingredients (API) with low solubility in aqueous media and use those enhanced API in a new approach of preparing modified and/or sustained release pharmaceutical formulation for API which are regularly not suitable for sustained release formulations because of their low solubility. This invention is applicable for small molecule API (molecular weight < 1000) for which the solubility can be enhanced by formation of a e.g., Cyclodextrin Complex regardless which Cyclodextrin or derivate thereof is employed according to the method described in this invention. Axitinib and other Kinase Inhibitors can be found for treatment of malign tumor diseases and others.

EP 4 729 049 A1

(52) Cooperative Patent Classification (CPC): (Cont.)

C-Sets
**C08L 5/16, C08L 1/284**

## Description

## FIELD OF THE INVENTION

The present invention is in the field of medicine, more in particular in the field of controlled release solid oral dosage forms for improved therapy. The dosage form according to the invention containing Axitinib or other kinase inhibitor can be used for treatment of different malign tumor diseases which are sensitive against kinase inhibitors, depending on the clinical profiling of the active pharmaceutical ingredients Axitinib or other kinase inhibitor. The present invention in particular discloses sustained release dosage forms for active pharmaceutical ingredient AXITINIB (API, AXT) with low solubility other kinase inhibitor with low solubility, wherein the API is incorporated into a complex for improved solubility and the said complex is used to form a sustained release dosage form.

## BACKGROUND

Controlled release dosage forms are key for the development of improved therapies, both through improved patient compliance and decreased incidences of adverse drug reactions. Controlled dosage forms may be used to delay absorption of a pharmaceutical active ingredient (API) until it has reached certain portions of the alimentary tract. Sustained release (SR) formulations are aiming to provide longer periods of pharmacologic action compared to periods obtained after administration of immediate-release (IR) dosage forms. They are further used to maintain a desired API concentration in the blood stream for a longer period than it would occur when conventional IR dosage forms are administered. Such longer periods of response provide for many therapeutic areas benefits which cannot be achieved with corresponding short acting, IR preparations. For example, therapy can be continued without interrupting the sleep of the patient. This is particularly important in the treatment of a patient with cardiovascular diseases or severe pain. Another advantage of longer acting drug preparations is the improved patient compliance resulting from the avoidance of missed doses through patient forgetfulness and improved convenience of application for the patient.

Unless conventional rapid acting medication is carefully administered at frequent intervals, peaks and valleys in the blood level of the API may occur due to rapid absorption, metabolic inactivation and systemic excretion of the compound, generating problems in therapy maintenance of the patient. Thus, it is a common goal in the field of pharmacologic research to develop sustained release dosage formulations that provide therapeutic concentrations of the API in blood which are maintained throughout the dosing interval, while the peak/trough concentration ratios are reduced. Hereby, drug development focusses on the many variables that influence the *in vivo* release and subsequent absorption of the active ingredients from the gastrointestinal tract.

Several prior art compositions exist which provide sustained release of pharmacologically active substances contained in the compositions after oral administration. These known sustained release formulations include specifically matrix formulations, coated formulations with diffusion membrane, osmotic systems and ion exchange resins. Formulations may be in form of pellets, tablets or capsules. In these formulations, the slow release of the active medicament is brought about through selective porosity of the coating, through a hole, or through compounding with a special matrix to affect the release of a drug. Some sustained release formulations provide related sequential release of a single dose of an active compound at predetermined periods after administration.

Controlled release compositions like quick-slow or delayed release formulations have constituted a clear advance in the art, particularly for medication available for conditions such as Attention Deficit Hyperactivity Disorder (ADHD), or for the treatment of insomnia, pain, depression and others.

Further described in prior art are multi-layer release (MLR) systems, comprising an outer immediate release (IR) layer, a release delaying layer (e.g., due to enteric coating) coated over the sustained release layer, and an IR core. These systems also avoid the manufacturing problems of blending multiple particle systems and have the advantage that the active ingredient is not released at once after the enteric coating is dissolved, due to the sustained release layer below.

In some of these multi-layer systems the release of the active ingredient also only starts once the enteric coating is dissolved, i.e., once the tablet has reached a specific pH in the gut. The point at which such pH is attained depends on each individual person, e.g., on what the patient has consumed before.

However, all those sophisticated modified release formulations have been used only for sustained/-modified/controlled release formulation of good soluble active ingredients.

Low solubility of APIs is frequently misinterpreted as providing intrinsic sustained release properties to a pharmaceutical dosage form. Therefore, SR formulations mostly are not used for low solubility API. If it is needed, e.g., to extent duration of action of an API, the development of a SR formulation of a less or low soluble API is regarded as easier compared to a good soluble API. Consequently, SR formulation principles for those APIs if any are only minimal adaptation of techniques which initially have been developed for good soluble APIs.

Sophisticated SR technologies known for good soluble API like diffusion matrix systems or complex diffusion membrane techniques for tablets or MUPS Systems are regarded as not suitable for low soluble API which is understandable because all these techniques are to a certain extent barriers for free dissolution of the API. Consequently, no SR products of low soluble API are marketed using a technique other than simple hydrophilic diffusion matrix systems, but none of these formulations are using cyclodextrin to enhance the solubility of the API initially.

During development of formulation with low soluble API, experts pay focus on enhancing solubility mainly by particle size reduction or co-precipitation with hydrophilic excipients including cyclodextrins. Development of SR formulation is considered as contradictory and adding complexity without creating advantages because sustained release techniques are regarded as "solubility lowering techniques". Avoidance of SR formulation is even more obvious because low solubility is frequently associated with long half-life of those API. At the end the question is why add a costly SR manufacturing process to a low soluble API formulation.

However, this approach neglects advantages of SR formulation other than just prolongation of time of action. A common observed mix-up of the distinction between the terms SOLUBILITY of an API and RELEASE of an API from a pharmaceutical dosage form may be another reason why SR formulations are rare for low soluble API.

As a conclusion no state of art SR formulation for low soluble API is available which provides similar advantageous properties as SR technologies known for good soluble APIs. This lack of available options is well understood because a SR technology is very likely to jeopardize the main challenge of a low soluble API development - enhancing its solubility.

The Concept of a state-of-the-art robust SR formulation - for soluble and as well as for low soluble API - should be independent of the solubility or particles size properties of the API to avoid intra- or inter patient treatment or stability issues because of multiple causes of variability.

Consequently, the following general conceptual sequence is guidance for the present invention.

A: First enhance the solubility of the API as much as possible with a reproducible and robust way - particle size reduction is not regarded as robust - and only if this is achieved:

B: Modify the drug release profile with a suitable pharmaceutical SR technology to get the desired property of the API in the pharmaceutical formulation like duration of action, bioavailability, site of absorption, steady state blood level, level of side effects and others.

Only following this approach, the known benefits of SR formulation are accessible for low soluble API as well without risking said disadvantages like variability or reduced bioavailability.

The present invention was fueled by the strong conviction that the same benefits which SR formulation offer to soluble API must be made available for less soluble API independent from its solubility properties. In this context It became obvious that advanced SR formulations technologies for low soluble API are not sufficiently available.

To overcome these difficulties, with API substances having a very poor aqueous solubility there have been attempts which used complexing of the API with cyclodextrins with the intention to enhance the solubility of the API. However, these attempts have not yet achieved efficient sustained release properties.

It is known that cyclodextrins form inclusion complexes with other molecules of suitable size and polarity (J. Pharm. Sci. 64, 1585, 1975). Cyclodextrins are cyclic compounds, consisting of 6, 7 or 8 glucopyranose units linked together by $\alpha$-1,4-glycosidic bonds. They are characterized by a cylindric structure and a special arrangement of hydroxyl groups, whereby the outer surface of the ring formed by cyclodextrin is hydrophilic, ensuring water solubility, whereas the inner surface is lipophilic, which permits other molecules, known as "guest molecules" or parts thereof, which are less polar than water (hydrophobic molecules) and are of suitable dimensions, to penetrate the lipophilic cavity of the inner part of the cylindric cyclodextrin molecule, forming thereby the inclusion complex.

For cannabinoids the solubility can be enhanced by dissolving the API in a triglyceride oil. In patent application US 2018/0250262 a formulation is disclosed comprising a solution of a cannabinoid combined with - among other excipients - triglyceride oil in form of sesame oil, cyclodextrin and high molecular weight hydroxypropyl methylcellulose (HPMC LCVR K100) without experimentally showing improved solubility or sustained release property. This formulation comprises components with conflicting functions, (i) triglyceride as solvent for the API but fatty oil at the same time is a target for complexation with cyclodextrin, (ii) triglyceride oil is as well a potential sustained release agent, (iii) questionable successful formation of a cannabinoid-cyclodextrin complex in the hydrophobic fatty oil environment. The combination of excipients does not allow prediction of the performance of a claimed sustained release property. Remaining Information from this example are known facts (i) cyclodextrin can be used to enhance solubility and (ii) hypromellose K100 is a suitable matrix forming sustained release agent; certainly not sufficient to start a new chapter of using cyclodextrin in sustained release formulations.

The present invention for the first time surprisingly provides evidence for improved solubility of a low-solubility API in purely aqueous media and secondly convert the solubility enhanced API into a sustained release formulation in which each component has a clearly defined function. The invention thus provides a reliably applicable sustained release formulation for Axitinib.

**BRIEF DESCRIPTION OF THE INVENTION**

The present invention relates to a solid oral pharmaceutical with a novel, well defined method to enhance solubility of active pharmaceutical ingredients Axitinib (API) with low solubility or other kinase inhibitor with low solubility in aqueous media above a pH of 3 and use of those solubility-enhanced API in a new approach of preparing modified and/or sustained/extended release pharmaceutical formulations for these APIs which are regularly not suitable for modified and/or sustained/extended release formulations because of their low solubility. This invention is applicable as well for other kinase Inhibitor active pharmaceutical ingredients (APIs) for which the solubility can be enhanced by formation of an API:cyclodextrin (CD) complex regardless which cyclodextrin or derivate thereof is employed according to the method described in this invention. Alternatively, other molecules apart from cyclodextrin may be used as complex forming agents instead of cyclodextrins depending on suitability for the API of interest. Alternatively, other media and pH-conditions during complexation may be used depending on specific properties of the API.

The present invention describes a two-step process wherein the first step is characterized by dissolving and/or mixing a low soluble API or a solution of the low soluble API with an aqueous solution of a suitable cyclodextrin /-derivate or another suitable complex forming agent. Applying the in-situ resulting-complex of a low soluble API with cyclodextrin/complex forming agent to a pharmaceutical acceptable carrier e.g., cellulose or sugar pellet while drying the solution/suspension, thus achieving a granule or pellet containing the solubility enhanced API complexes. In the second step of the invention the solubility enhanced API is converted into a SR formulation by applying SR techniques which are known for good soluble API only. This step is regarded critical because it has not been investigated if the API-cyclodextrin/complex forming agent complex remains stable in sustained release formulation and does not prematurely dissociate prior being released from the sustained release formulation during in-vitro dissolution testing and consequently before reaching the in-vivo absorption site. During the development of this invention, surprisingly it has been found that by applying a sustained release technology the entrapment efficiency of the API inclusion complex is enhanced because it prevents spontaneous dissociation of the API-complex. This enables the use of the technology of this invention for the complexation of low soluble API with cyclodextrin despite the common issue that the complex may dissociate in the larger volume of media in the use environment like dissolution testing media or in-the GI tract. Thus, API-complexes according to this invention, even with low entrapment stability in aqueous media, may become suitable targets for sustained release formulations.

In this respect it has surprisingly been found that pellets coated in the first step with the solubility enhanced API-CD complex are stable and did show in-vitro SR properties after being coated with a diffusion membrane, which is regarded as the most critical SR technique in this context, i.e., the SR formulation which presents the most challenging technical requirements for adaptation to low solubility API. Diffusion membrane SR formulations require movement of the API across a membrane with defined pore sizes for API release. In contrast to other SR formulation technologies only the diffusion membrane SR technology is a solid physical barrier controlling diffusion of API or API-cyclodextrin complex according to this invention through hydrophilic pore builder incorporated in the membrane. Before the present invention it was unclear if such diffusion membrane SR formulations can be adopted for API:CD complexes of low solubility API or if release of API from the API:CD complexes would be detrimentally inhibited by the diffusion membrane. This application presents for the first time examples for Axitinib or other kinase inhibitor in a diffusion membrane multi-particle SR formulation. However, the same principle of the invention, i.e., combining a solubility-enhanced API complex with a SR formulation, can also be applied and transferred to other sustained release formulation techniques like aqueous (hydrophilic) diffusion or erodible matrix, non-aqueous fatty acid matrix, melt extrusion system, osmotic pump systems and others. It is expected that the API-cyclodextrin complex will not disintegrate in other SR technologies either and SR formulation employing these techniques will show SR properties in vitro as well as in-vivo.

Further, other embodiments of the invention may combine the SR formulations of the solubility-enhanced API with an IR (immediate release) formulation techniques to prepare a modified or dual phase release pharmaceutical dosage form.

The present invention hereby discloses a novel method of an effective preparation of solubility enhanced low soluble API by formation of an API/CD complex without the requirement of isolation of the intermediate and sophisticated physical characterization of the properties of the obtained solubility enhanced API:CD complex, which subsequently are employed in techniques to prepare modified and/or sustained release formulation systems.

In one aspect of the invention, the invention relates to a solid pharmaceutical composition for oral administration comprising at least one active pharmaceutical ingredient (API) in a noncovalent inclusion complex with a complex-forming agent, wherein the API-inclusion complex is applied to a pharmaceutical carrier and wherein the pharmaceutical carrier carrying the API-inclusion complex are formulated as a sustained release formulation. The inclusion complex in the meaning of the present application is "guest molecule" enclosed in a complex-forming agent such as cyclodextrins without forming covalent bonds between these entities. The inclusion complex may include more than one molecule of either the "guest molecule", i.e., the API, or more than one molecule of the complex-forming agent depending on the mixing ratio and size ratios.

In a preferred embodiment of the invention API-inclusion complex is first applied to a pharmaceutical carrier and wherein the pharmaceutical carrier carrying the API-inclusion complex are subsequently formulated as a sustained release formulation.

In contrast there are also hydrophobic solubility enhancing agents which include in particular fatty oils, triglycerides and other lipophilic and hydrophobic solvents. In a preferred embodiment of the invention no hydrophobic solubility enhancing agents are added to the composition.

A second aspect of the invention relates to a process for preparing the pharmaceutical composition according to the invention comprising the steps: (i) preparation of an API-inclusion complex comprising an API with low solubility and a complex-forming agent, and (ii) preparation of a sustained release formulation comprising the API-inclusion complex of step (i).

A third aspect of the invention relates to a process for preparing the pharmaceutical composition according to the invention wherein the dissolution profile of the pharmaceutical composition refers to the release of the solubility-enhanced API and the release profile of sustained release formulation achieves a maximum in-vitro release (MDR) in aqueous media of 70% of the cumulative finally released solubility-enhanced API within 1 h as determined by in-vitro dissolution testing in aqueous media and a release rate of greater zero in more than 2 consecutive time periods of 30 minutes as determined by in-vitro dissolution testing in aqueous media.

A fourth aspect of the invention is a method to evaluate an in vitro dissolution profile of any one of the pharmaceutical composition according to the invention, wherein the dissolution profile is generated in aqueous media to determine the entrapment efficiency and stability of an API-complex, wherein the API-inclusion complex comprises an API with low solubility in an aqueous solution and a complex-forming agent, wherein the API-inclusion complex is applied to a pharmaceutical carrier, wherein the pharmaceutical composition comprising the API-inclusion complex is formulated as a sustained release formulation.

**DEFINITIONS**

Unless otherwise defined, all technical and scientific terms used herein have the same meaning as commonly understood by one skilled in the art to which this technology belongs. Although all methods and materials similar or equivalent to those described herein may be used in practice or in testing the present technology, the preferred methods and materials are now described.

In the context of the present invention, the term "chemical library" refers to a collection of distinct and variable chemical entities, wherein these compounds may be selected from any category of compounds and show large variety of different properties. The library contains compounds organized in an ordered way and may contain hundreds, thousands, millions, or billions of different substances. The chemical library of the invention may be a library of suitable low solubility API according to the present invention.

As used herein, the term "chemical substance/-compound" refers to any material with a definite chemical composition and comprises any organic or inorganic substance of a particular molecular identity and may exist in different physical states such as a liquid, a solid or gas. Examples comprise but are not limiting to alcohols, aldehydes, esters, terpenes, aromatics, ketones, lactones, thiols, hormones, amines, siderophores, acids, antimicrobials, fungicides, toxins. A chemical substance/compound may also refer to antibodies, nanobodies or peptides or generally the API of the present invention.

In the description of the methods of the present invention the use of terms for individual steps of the methods such as "first", "second"; "i.", "ii" or "a", "b" etc. does not necessarily imply a required succession of steps. Depending on the method and required time intervals some of these steps may occur simultaneously or in different orders than the listed succession.

In the context of this invention "agitate" or "agitation" refer to various mechanical methods of mixing of different substances which may include, but are not limited to, rotating, vibrating, vortexing, swirling, shaking, ultrasonicating, stirring, or any movement that causes mixing. Mechanical movements include movements performed by hand or by a rotator.

The present invention refers to different substances which are included into SR dosage form as "Active Pharmaceutical Ingredient" (API). An API is defined as: A biologically active ingredient in a finished product having a direct effect in the diagnosis, cure, mitigation, treatment or prevention of a disease, or in restoring, correcting or modifying one or more physiological functions in a subject, such as a human or animal subject.

Depending on the API and the dosage form, the API may be administered in form of a solution. Hence, at least one solvent is required which may be combined with further "co-solvents", i.e., a solvent added to a fluid in an amount less than 50% of the total volume.

For improving the solubility of hard to dissolve API different means are available. In the context of this invention "cyclodextrins" can be used to form API-cyclodextrin inclusion complexes with improved solubility. Cyclodextrins are a family of cyclic oligosaccharides produced from starch by enzymatic conversion wherein cyclodextrins have a structure comprising a macrocyclic ring of α-D-glucopyranoside units joined by α-1,4 glycoside bonds. Typical cyclodextrins contain six to eight glucose subunits in a ring, creating a cone shape. α-Cyclodextrin contains six glucose subunits; β-cyclodextrin contains seven glucose subunits; and γ-cyclodextrin contains eight glucose subunits. Because cyclodextrins have an inner hydrophobic core and a hydrophilic exterior, they form complexes with hydrophobic compounds such as the low solubility APIs of the present invention in preferably a 1:1 or 1:2 molecular ratio of API and Cyclodextrin.

The SR formulation of the invention contains and provides an "effective amount" of the API, wherein an effective amount is understood as the amount of an active agent (API) (alone or with one or more other active agents) sufficient to induce a desired response, such as to prevent, treat, reduce and/or ameliorate a condition.

The SR of the invention may contain one or more "emulsifiers". Emulsifiers are commonly understood as surfactants which reduce surface tension between oil and water phases. Thus, emulsifiers facilitate formation of globules due to reduced surface energy. Emulsifiers include gums, fatty acid conjugates and cationic, anionic and amphoteric surfactants capable of suspending the oily phase and stabilizing the emulsion by coating the oil droplets and avoiding the separation of the internal oily phase. The film coat produced by the emulsifier further provides a barrier between the immiscible phases and therefore prevents droplet association, coagulation and coalescence. Examples of emulsifier include, but are not limited to, lecithin, glyceryl monostearate, methylcellulose, sodium lauryl sulfate, sodium oleate, sorbitan monopalmitate, sorbitan monostearate, sorbitan tristrearate, tragacanth, triethanolamine oleate, polyethylene sorbitan monolaurate, poloxamer, detergents, Tween 80 (polyoxyethylene sorbitan monooleate), Tween 20 (polyoxyethylene sorbitan monolaurate), cetearyl glucoside, polyglucosides, sorbitan monooleate (Span 80), sorbitan monolaurate (Span 20), polyoxyethylene monostearate (Myrj 45), polyoxyethylene vegetable oil (Emulphor), cetyl piridinium chloride, polysaccharides gums, Xanthan gums, Tragacanth, Gum arabica, Acacia, or proteins and conjugated proteins capable of forming and protecting stable oil in glycerin emulsion. The expert in the field is well familiar with suitable emulsifiers and the specific requirements for selecting an emulsifier for a given application.

Solubility of the API is for many substances significantly affected by the pH of the solution. Therefore, the SR of the invention may include one or more "pH adjusters" or "pH modifiers". A pH adjuster or modifier is a molecule or buffer used to achieve necessary pH control and to adjust a specific target pH in a formulation. Exemplary pH modifiers include acids (e.g., acetic acid, adipic acid, carbonic acid, citric acid, fumaric acid, phosphoric acid, sorbic acid, succinic acid, tartaric acid), basic pH modifiers (e.g., magnesium oxide, tribasic potassium phosphate), and pharmaceutically acceptable salts thereof or combination of acid and salts forming a buffer.

Preparation of the SR formulation of the invention may further include "purification" of the API or other components. Within the context of the present invention purification includes any technique or method that increases the degree of purity of a substance of interest, such as an enzyme, a protein, or a compound, from a sample comprising the substance of interest. Said methods include but are not limited to mixing with charcoal, silica gel column chromatography, size exclusion chromatography, hydrophobic interaction chromatography, ion exchange chromatography including, but not limited to, cation and anion exchange chromatography, free-flow-electrophoresis, high performance liquid chromatography (HPLC), and differential precipitation. Said methods of purification achieve an improved "purity" of the substance, i.e., a quality of an unadulterated, uncontaminated and safe product obtained by the disclosed methods and meeting the necessary pharmaceutical standards.

Solubility may further be affected by "viscosity", i.e., the measure of a fluid's resistance to gradual deformation by shear stress or tensile stress.

A substance of composition which is "water-immiscible" refers to any non-aqueous or hydrophobic fluid, liquid or solvent which separates from solution into two distinct phases when mixed with water.

A substance or compound which has "low aqueous solubility" according to the invention refers to a compound or composition having a solubility in aqueous media (water, aqueous acid or basic solutions or puffer with pH between 0.05 and 12), of less than 10 wt%, less than 5 wt%, less than 3 wt%, or less than 1 wt%, measured in water or another appropriate aqueous media between 4 and 37 °C at buffer pH 6.8, preferably at 20°C at buffer pH 6.8. Alternatively the substance or compound which has "low aqueous solubility" according to the invention refers to a compound or composition having a solubility in aqueous media (water, aqueous acid or basic solutions or puffer with pH between 0.05 and 12), of between 5 to 10 wt%, between 3 to 5 wt%, between 1 to 3 wt%, between 0.1 to 1 wt% and between 0.01 to 0.1 wt%, and between 0.1 to 3 wt%, between 0.1 to 5wt%, between 0.1 to 10 wt% and between 1 to 5 wt%, between 1 to 10 wt% and 3 to 10 wt%, measured in water or another appropriate aqueous media between 4 and 37 °C at buffer pH between 0.05 and 12, preferably between 4 and 37 °C at buffer pH 6.8, more preferably at 20°C at buffer pH 6.8. Appropriate in this context means pH conditions in which the low soluble substance is not converted in another polymorph, e.g. a Hydrochloride-salt of a substance will be converted into free base or a free base may be converted into a salt in acid media. Methods to determine solubility in context of this application are described in section "Detailed description of the Invention". Within the meaning of this application a substance or compound with low aqueous solubility is determined by following standard methods as defined in US Pharmacopeia standard (chapter <1236> "Solubility Measurements"). The methods and conditions for determining solubility are selected by the skilled person based on common knowledge and as appropriate for the tested substance or compound.

"Sustained release", "retarded release", or "prolonged release" are used synonymously within the present application. These terms mean that the dosage form releases no greater than about 70 wt% of the API in the dosage form during the first hour and displays a release rate of greater zero in at least 2 consecutive time periods of 30 minutes after administration to a use environment. As used herein, a "use environment" can be either the in vivo environment, such as the G! tract of an animal, particularly a human, or the in vitro environment of a test solution, such as 0.1 M HCl gastric fluid (GF), phosphate buffered saline (PBS) solution, Model Fasted Duodenal (MFD) solution, or a simulated intestinal buffer solution at various pH values. Thus, the dosage form may release the API gradually and continuously over a release period, may release the API in a pulsatile or delayed manner, or may release the API in a combination of release profiles, such as an immediate release burst followed by either a delayed burst or by a gradual and continuous release.

In accordance with standard understanding in pharmacology "sustained release", "retarded release", or "prolonged release" implies release over a prolonged period of time in comparison to an immediate release form, wherein the drug release onset is rapid and the MDR (maximum released drug) is reached after a short period. In contrast "controlled release" implies drug release over a prolonged period indicated by a positive (greater than zero) release rate. Both concepts are summarized as "extended release" dosage forms. Finally, "delayed release" implies a dosage form which releases the API after period of delay wherein no or nearly no release of the API is observed, typical examples are dosage forms which contain enteric coatings which achieve pH-dependent delay that ensures release of the API is delayed, e.g., until after the dosage form has passed the stomach. Thus, "delayed release" forms are used to control the site of drug release in contrast to "extended release" forms which primarily control the time period of drug release. All these concepts are collectively understood as "modified release" forms in direct contrast to "immediate release" forms.

"Administration to a use environment" means delivery of the API into the above defined use environment by appropriate means. Therefore, in case of an in vivo use environment, e.g., the G! tract, administration implies delivery by ingestion or swallowing or other such means to deliver the dosage form. Alternatively, in case of an in vitro use environment, administration refers to placement or delivery of the dosage form to the in vitro test medium.

**FIGURE CAPTIONS**

Figure 1

Comparison of the dissolution of solubility enhanced Axitinib using CD complex (experiment 1) vs. dissolution of Axitinib without enhanced solubility (experiment 2) as determined by in-vitro dissolution test assay. The test was performed in buffer pH 6.8 under "non sink" conditions, meaning that the tested amount of Axitinib is not fully soluble in the amount of dissolution media. In addition, the Axitinib:CD complex is partly dissociating because of the altered complexation balance in the large amount of dissolution media. Despite these unfavorable experiment conditions, the solubility of Axitinib:CD complex is increased from 1.25 $\mu$g/ml to more than 5 $\mu$g/ml (about 4-fold) compared to a composition with a regular binder (HPMC 603) only.

After adding an overage of HP-R-Cyclodextrin after 120 Minutes of testing to achieve dissolution "sink" conditions the steep increase of the dissolved amount of Axitinib - suggest that solubility enhancement is even higher.

Figure 2

Comparison of release profiles of solubility enhanced Axitinib:CD IR Pellets with those Pellets coated with different SR release diffusion membrane coating composition.

The results show that the solubility enhanced property of the solubility enhanced Axitinib pellets is retained after sustained release coating. The release property of the SR pellets is displaying a typical sustained release characteristic, and the release time is depending on the amount and composition of sustained release coating applied to the pellets containing solubility enhanced Axitinib. The cumulative release amount is in all instances higher than the release of pure Axitinib in the same dissolution media.

**DETAILED DESCRIPTION OF THE INVENTION**

The present invention refers to small API molecules with low solubility in aqueous media, in particular Axitinib or other kinase inhibitors. Commonly for such APIs only the low solubility is exploited to provide the effects of a sustained release formulations, i.e., sustained release is a result of the slow dissolution of the API in place. This approach entails certain disadvantages as the actual rate of dissolution cannot be accurately controlled and adjusted and therefore is subject to significant individual variability. Such variability and therefore uncertainty in API dissolution is not desirable as the pharmacokinetic of the API is inconsistent. Further, this approach significantly limits the achievable effective concentrations of the API during therapy, particularly in a sustained release formulation.

This invention presents a solution to the above problem, by first addressing the low solubility of the API, which is incorporated in an inclusion complex to increase the solubility as much as possible. Subsequently, said inclusion complex is used to produce a sustained release dosage form of the API with a well-defined and reproducible release characteristic which is providing less inter- and intra-individual variability. The sustained release dosage forms of the complexed API of the invention provide stable, non-degradable, edible, dispersible or drinkable compositions comprising pharmaceutical grade encapsulated API to produce the highly bioavailable encapsulated API of pharmaceutical grade purity. The disclosed quick, cost-effective, and easily scalable manufacturing processes does not require the use of toxic organic solvents and thus satisfy the most restrictive health guideline requirements for residual solvents. The resulting API dosage form may be used for pharmaceutical oral delivery, food production and medical applications. In addition, the enhanced solubility of the API means that higher therapeutic concentrations may be achieved during therapy or enables reducing of the administrated individual dose without effecting the bioavailability but reducing potential side effects.

The solution of the present invention involves the synergistic combination of firstly increasing the solubility of the low solubility API, wherein the API is incorporated into an API-inclusion complex. This means that the solubility of the API is enhanced without chemical modification of the API molecule or physical particle size reduction of the API. Instead, API solubility is enhanced by physical means on a molecular level. API solubility enhancement is measured as cumulative in-vitro dissolution (MDR) in common dissolution apparatus in aqueous media, wherein the API-inclusion complexes show a significantly increased solubility compared to pure or regular processed API. After preparing API-inclusion complexes with enhanced solubility the invention further relates to a second step of forming modified/slow/delayed/extended/sustained release formulations which contain the API inclusion complexes, wherein the present invention preferably refers to sustained release forms. The dissolution of the SR formulations containing the solubility enhanced API is also measured overtime (cumulatively=MDR) in in-vitro dissolution test apparatus according to US Pharmacopeia standard (chapter <711> "Dissolution") in aqueous media, wherein again the solubility of the API-inclusion complexes as released from the SR formulations is significantly higher than pure or regular processed API.

In the following detailed description of sustained release (SR) formulations containing solubility-enhanced API according to the invention, different alternative embodiments are presented, which may or may not be used depending on the API and the therapeutic requirements. Hence, all below described alternative embodiments are to be understood as a selection of options from which a specific SR formulation may be selected by combining various of the presented options.

The inventor surprisingly found that for small low solubility API the combination of a solubility-enhanced API with a sustained release (SR) formulation according to the invention, consisting of specific hydrophilic or hydrophobic matrix forming polymers and excipients (Matrix Formulation), specific compositions forming an osmotic pump (OROS system), specific coatings and layers in optimized orders on tablets or pellets (diffusion membrane formulation) and comprising at least one pharmaceutical active ingredient, a very defined and controlled release of the at least one active pharmaceutical ingredient (API) can be achieved. This novel SR formulation results in specific and beneficial pharmacokinetics that contribute to the benefit of the patient having to be treated with one or more APIs. The dosage form of the invention can be applied to a broad range of APIs and consequently has many indications.

Suitable API are characterized by their low solubility in aqueous solutions and their sufficiently small molecular size which allows inclusion into the inclusion complexes (generally smaller than 1000 Da). The API of the present application is Axitinib or other kinase inhibitors.. Solubility of API in aqueous solutions is determined by in vitro dissolution test as defined in US Pharmacopeia standard (chapter <711> "Dissolution") with apparatus 1 or 2 of their proposed apparatus and conditions; e.g., 37 °C and basket (apparatus 1) paddle rotation (apparatus 2) of 100 or 50 rpm, respectively, with either water or standard aqueous media at pH 1, pH 2, water or buffer pH 6.8 according to US Pharmacopeia standard (chapter <711> "Dissolution"). A different aqueous media may be selected if required for stability reasons of the API; e.g., decompensation of the at pH 6.8 or change of the polymorph of the API, e.g. salt formation of a base under low pH conditions and vis versa base formation of API with a strong acid at higher pH values. Preferably aqueous conditions are selected to maintain an unchanged API polymorph. The amount dissolved is determined either by measuring the concentration at the defined time points and the amount dissolved is plotted versus time. The amount at each time point is determined by a suitable assay method, e.g., UV for API with sufficient extinction coefficient or HPLC with low extinction coefficient. The cumulative released amount result is calculated as % of the tested dose of the API over a period of at least 180 minutes.

In some embodiments, the disclosed SR compositions may include, but are not limited to capsules, tablets, pills, powders, beads, lozenges, sugar coated tablets, granules, dietary compositions, food products, beverages, emulsions, solutions, suspensions, suppositories, creams, gels, syrups, elixirs.

In preferred embodiments, the oral dosage form is a capsule. In another preferred embodiment, the oral dosage form is a tablet or pellets or tablets and capsules containing pellets or granules (MUPS).

**Solubility-enhanced low solubility API**

As stated above the first aspect of the present invention is enhancing the solubility of the small API with low solubility in an aqueous media. Different techniques and approaches for increasing solubility for different chemical classes of API are known. Hence, in some embodiments of the invention different solubility enhancing agents are used to provide a solubility enhanced API according to the invention. The solubility enhancing agent include complexing agents and may include one or more surfactant, solubilizer, hydrophilic agent, pH modifying agent, and the like. The solubility enhancing agent could be the same or different for different API.

The APIs of the present invention is Axitinib or other kinase inhibitors, which have low solubility within the meaning of the present invention.

Enhancement of solubility of the API may be in particular achieved by forming inclusion complexes of the API and a suitable complex forming agent. The complexing agent includes, but is not limited to, cyclodextrin class of molecules, such as cyclodextrins containing from six to twelve glucose units, especially, α-cyclodextrin, β-cyclodextrin, γ-cyclodextrin, or their derivatives, such as hydroxypropyl-alpha-cyclodextrin, methyl-beta-cyclodextrin, hydroxypropyl beta cyclodextrins, sulfobutyl-ether-beta-cyclodextrin, hydroxypropyl-gamma-cyclodextrin or mixtures thereof. In this complexation process, a hydrophilic polymer may be additionally added to further enhance the solubility along with the complexing agent.

For preparing the solubility-enhanced API according to the present invention the solubility enhancing agent and the API may be employed in different ratios. The selected ratio depends upon the desired and required molecular ratio between the cyclodextrin and the API and the stability of the resulting inclusion complex related to the type of further solubility enhancing agents employed and the API of interest. Within the scope of the invention the ratio of API to solubility enhancing agents may range from about 1:1 to about 1:5 and preferably the ratio is about 1:1 to about 1:2 with 10% overage of the solubility enhancing agent. A combination of solubility enhancing agents may also be included where the total amount of solubility enhancing agent employed is maintained in the above-mentioned ratios.

In a preferred embodiment of the invention the solubility enhancing agent is a complex-forming agent, wherein different molecules are known as possible complex-forming agents. In a more preferred embodiment, the complex forming agent is a cyclodextrin. Cyclodextrins are frequently used and of particular suitability as complex forming molecules. Cyclodextrins (CDs) are cyclic oligosaccharides consisting of (α-1,4)-linked α-D-glucopyranose units that contain a lipophilic internal cavity and a hydrophilic outer surface resembling the shape of a truncated cone. The hydroxyl functions of the glucose are orientated to the exterior of the cone, where primary hydroxyl groups are in the narrow edge of the cone and the secondary hydroxyl groups at the wider edge. The inner cavity is lined by skeletal carbons and ethereal oxygens of the sugar residues, which gives it a lipophilic character. These characteristics allow CDs to form inclusion complexes with a variety of host-guest molecules of suitable polarity and size. Their use has been extensively exploited to improve the pharmaceutical properties of numerous drugs such as water solubility, chemical stability, avoid physicochemical incompatibilities, oral absorption and to modulate biological activity. Pharmaceutically well accepted cyclodextrins include, but are not limited to, α-cyclodextrin, β-cyclodextrin and γ-cyclodextrin and its derivates, ether-derivates of α-cyclodextrin, β-cyclodextrin and γ-cyclodextrin including hydroxypropyl-α-cyclodextrin, hydroxypropyl-β-cyclodextrin, hydroxypropyl-γ-cyclodextrin, methyl-α-cyclodextrin, methyl-β-cyclodextrin, methyl-γ-cyclodextrin, sulfobutyl-α-cyclodextrin, sulfobutyl-β-cyclodextrin, sulfobutyl-γ-cyclodextrin are pharmaceutically suitable to be used individually or as mixture in an embodiment of the invention. Other hydrophilic derivatives like acetylated forms of cyclodextrin include, but are not limited to, α-cyclodextrin exadeacetate (AACD), β-cyclodextrin heneicosaacetate (ABCD), and γ-cyclodextrin octadeacetate (AGCD), and other suitable hydrophilic α-cyclodextrin, β-cyclodextrin and γ-cyclodextrin derivatives and any mixture thereof or mixtures with other Cyclodextrins are within the scope of this invention.

Beta cyclodextrin (β-CD)-based nanosponges (NS) are biocompatible and nanoporous carriers having the capability of forming supramolecular inclusion complexes as well as non-inclusion complexes with both hydrophilic and lipophilic drugs. Toxicological studies of the NS have proven them to be safe for use in pharmaceutical products.

For example, hydrophilic CD derivatives such as 2-hydroxypropyl-β-CD and sulfobutylether of β-CD are useful for improving solubility and dissolution rates of poorly water-soluble API, while hydrophobic CD derivatives such as ethylated and acylated CDs can work as slow-release carriers for water-soluble drugs.

Other complex-forming agents for formation of API inclusion complexes for enhancing of API solubility are known. Hence, in an alternative embodiment Chitosan is used as complex-forming agent. Chitosan [(1-4)-2-amino-2-deoxy-β-d-glucan] is a cationic polysaccharide of natural origin obtained by N-deacetylation of chitin, resulting in a copolymer of N-acetyl-d-glucosamine and d-glucosamine. Chitosan has high biocompatibility, biodegradability and lack of toxicity associated with bioadhesiveness, dissolution, and permeation enhancer properties.

Furthermore, its antiacid and antiulcer activities can be exploited to reduce gastric irritation caused by certain API, such as anti-inflammatory drugs. Finally, a synergistic effect of cyclodextrin and chitosan in improving solubility and/or permeation properties of some drugs has been observed, indicating such a polymer as a good potential partner of cyclodextrins. Thus, in another alternative embodiment the complex-forming agent may also be a mixture of cyclodextrins and chitosan, including possibly chemically modified forms of either cyclodextrins and chitosan for optimized complex formation with the selected API by adjusting polarity, hydrophilicity and hydrophobicity of the complex-forming agents.

In some embodiments of the invention the formation of the API-inclusion complexes may be achieved by combining the API, extracts, distillates, refined distillates, twice-refined distillates, three time-refined distillates or high quality isolates with acetylated and/or hydrophilic cyclodextrins, unmodified cyclodextrins, chitosan or other complex-forming agents in API-complex-forming agent molar ratios ranging from 1:0.5 to 1:5. In some examples, the API:cyclodextrin molar ratios are 1:0.75, 1:1, 1:1.5, 1:2, 1:2.5, 1:3, most preferred molar ratios of API:cyclodextrin are 1:1 and 1:2. In addition an overage may be required to stabilize the equilibrium of the API: Cyclodextrin complex during production process, analytical testing or passage through the intestine. The overage may add 10%, 15%, 20%, 30% or 40% additional cyclodextrin to the above listed ratios. This means that including the overage an API:cyclodextrin mixing ratio of, e.g., 1:1 is to be understood as ranging between 1:1 and 1:1.1; 1:1.15; 1:1.20; 1:1.30 or 1:1.40 depending on cyclodextrin overage. The overage of cyclodextrin in relation to the API concentration leads to an improved stability and reproducibility of API:cyclodextrin complex formation during processing of the APkcyclodextrin solution which is affected less by other factors.

**Sustained release formulations for solubility-enhanced API**

The invention also relates to sustained release (SR) pharmaceutical preparations and formulations, comprising a therapeutically effective amount of the solubility enhanced API-inclusion complex together with a pharmaceutically acceptable carrier and other common excipients and further functional elements as described below.

Pharmaceutical SR compositions are preferably formulated in forms suitable for oral application, such as tablets, capsules, granules or other oral preparations. At formulating, it should be taken into consideration that the content of the API or a pharmaceutically acceptable salt in the final dosage is in the range of 0.1 - 90%, from 1 to 70%, from 1-50%, from 2 to 80%, from 2 to 50% from 5 to 80%, from 5 - 50 %, from 10 to 80 %, from 10 to 59 % and the content of thereof in the solubility enhanced API complex is in the range from 0.2% to 80%, from 1% to 80%, from 2% to 80%, from 10% to 60%, from 10% to 50%, from 10% to 40%, or from 10% to 30%.

The pharmaceutical SR composition containing the resulting solubility enhanced API-complex is administered orally, wherein the complex forming molecule, i.e., cyclodextrin etc., functions exclusively as an agent for enhancing solubility of the API and does not alter the therapeutic mode of action of the API in any way. Oral SR pharmaceutical preparations (tablets, capsules, etc.) are prepared by incorporating the inclusion complex of the API with the complex-forming molecule, as cyclodextrins etc., into a SR technology, e.g., hydrophilic or hydrophobic matrix, osmotic pump, diffusion membrane etc which releases the API-inclusion complex in a controlled way by diffusion, and/or slow erosion of the matrix, osmotic pressure or by diffusion through a permeable membrane.

The central consideration of a sustained release (SR) dosage form or formulation is the intended time frame in which release of the API is extended compared to an immediate release (IR) formulation. A SR formulation is characterized and distinguished compared to an IR formulation by the (a) the time the maximum dose (MDR) is released and (b) the slope of the in-vitro cumulative measured amount of drug release from the formulation. The MDR from an IR formulation is limited by the solubility of the API in the formulation and widely expected within the first hour. This type of formulation form can thus be characterized by a slope greater than 0 for two 30 minutes-intervals only in a dissolution test performed for 1 hour.

In contrast a minimal but widely accepted definition for SR formulation is characterized by (a) a slope of greater than 0 for more than two consecutive intervals of at least 30 minutes at any of the measured cumulative dissolution test (b) a MDR later than 1 hour and (c) MDR at 1 hour of less than 70% of the overall MDR in the given dissolution test of at least 2 hours. This definition is applicable for all subtypes of SR formulation known in the art e.g., delayed release, controlled release, quick slow release (with maximum 70 % IR portion). The overall time until MDR is achieved may vary between 2 and 72 hours.

IR and SR formulation prepared following the techniques described and tested for dissolution according to the apparatus and conditions described in US Pharmacopeia standard (chapter <711> "Dissolution") are in the scope of this invention when the results are meeting the above provided definitions.

Some pure API may show slow dissolution kinetic suggesting intrinsic SR properties. This property is outside of the scope of this invention.

Thus, the SR formulations according to the present invention have a maximum in-vitro release (MDR) in common dissolution tests according to US Pharmacopeia standard (chapter <711> "Dissolution") in aqueous media of 70%, preferably 65%, more preferably 60% and most preferably 55% of the declared SR portion of the cumulative finally released APIs within 1 h and the maximum released amount of the solubility-enhanced API complex, depending on the desired duration of the SR effect, is reached in up to 48 h, in 36 h, in 24 h, in 20 h, in 16 h, in 12 h, in 10 h, in 8 h, in 6 h, in 4 h, in 3 h or in 2 h.

In an embodiment of this invention the SR formulated solubility enhanced API can be formulated further comprising an IR portion of the same or more than one API in a single dosage form in which a minimum of 50 % of the cumulative in-vitro released IR portion of the API is released within 1 hour. In this and comparable cases, the above provided definition for IR and SR are applicable to the respective parts only.

In another embodiment the sustained release formulation of the present invention is an oral dosage form comprising more than one layer, for example 2 or 3 layers. In a preferred embodiment, the oral dosage form comprises a bi-layer core, comprising an active layer and a sweller layer. The core may be coated. The oral dosage form comprising multiple layers may, in one embodiment, comprise one or more holes on the surface of the coating on the active layer side forming an osmotic pump system (OROS).

Thus, for example multi-layered SR formulations can be designed for the solubility-enhanced API of the invention. Therefore, in one aspect, the invention relates to a solid oral pharmaceutical dosage form comprising a SR formulation and at least one API or a pharmaceutical acceptable salt, an isomeric form, a prodrug, or metabolite thereof, wherein the API or its derivative are provided in a solubility enhanced preparation. The solid oral pharmaceutical SR dosage form may further comprise other features and layers depending on the API and the required therapy and dosage regiment. These further elements are selected from, but not limited to, an immediate release active core, a delayed-release layer comprising an enteric or delayed release coating, a sustained release layer comprising a retard polymer, and/or an immediate release layer comprising an active pharmaceutical ingredient. Hence, by combination of sustained/delayer release layers with immediate release layers the dosage formulation of the invention may exhibit a bi-modal release profile of the at least one active pharmaceutical ingredient. This layered formulation may be used such that upon oral administration, the formulation provides a rapid dissolution and absorption of the outer layer of the formulation which contains a portion of the API in immediate release form, thereby resulting in a rapid rise of the drug to therapeutic plasma levels. This is followed by a period of controlled release of the drug from the formulation. If present in the SR formulation an enteric coating delays the absorption of the main portion of the API until a point a pH is attained for the enteric coating to be dissolved. That way, the dosage form is able to mitigate the variability of the start of the release caused by the pH variability in the gut.

In further embodiments the SR formulations according to the invention are intended to be administered in specific dosage regiments, those SR formulations may be administered with intervals between dosages of more than a day or preferably once a day or most preferably twice daily. The sustained release dosage forms may provide greater convenience and compliance due to improved ease of use for the patient. However, the total passage time through the intestine may limit the extension of administration intervals because of specific condition required at the site of use.

Depending on the selected API and the solubility-enhanced API-complex some embodiments of the present sustained release dosage forms should be administered with or without food to further facilitate efficient uptake. For some APIs absorption of the API may be significantly increased when the API is administered with food and vis versa.

There are different means known in the field for achieving different types of SR formulations for the solubility-enhanced API according to the invention. The present invention refers to any available SR formulation which is suitable for the API of interest and fulfils the therapeutic requirements and which is suited to the mode of administration. Below different embodiments of available sustained release formulations are described by their constituent components, in many cases it is possible to combine certain elements such as matrix materials, coatings or other listed excipients of different embodiments.

Matrix Sustained Release Formulations

Firstly, SR formulations may be achieved by using Matrix Sustained Release Dosage Forms.

Matrix Sustained Release Dosage formulations are characterized by incorporating the solubility-enhanced API into an erodible or non-erodible polymeric matrix. An erodible matrix is understood in the filed as aqueous-erodible or water-swellable or aqueous-soluble in the sense of being either erodible or swellable or dissolvable in pure water or requiring the presence of an acid or base to ionize the polymeric matrix sufficiently to cause erosion or dissolution. When contacted with the aqueous use environment, the erodible polymeric matrix imbibes water and forms an aqueous-swollen gel or "matrix" that entraps the solubility-enhanced API. The aqueous-swollen matrix gradually erodes, swells, disintegrates, disperses or dissolves in the environment of use, thereby controlling the release of solubility-enhanced API to the environment of use. Examples of such dosage forms are well known in the art.

The erodible polymeric matrix into which the solubility-enhanced API is incorporated may generally be described as a set of excipients that are mixed with solubility-enhanced API that, when contacted with the aqueous environment of use imbibes water and forms a water-swollen gel or "matrix" that entraps the API. API release may occur by a variety of mechanisms: the matrix may disintegrate or dissolve from around particles or granules of the API; or the API may dissolve in the imbibed aqueous solution and diffuse from the tablet, beads or granules of the dosage form. A key ingredient of this water-swollen matrix is the water-swellable, erodible, or soluble polymer, which may generally be described as an Osmo - polymer, hydrogel or water-swellable polymer. Such polymers may be linear, branched, or crosslinked. They may be homopolymers or copolymers. Although they may be synthetic polymers derived from vinyl, acrylate, methacrylate, urethane, ester and oxide monomers, they are most preferably derivatives of naturally occurring polymers such as polysaccharides or proteins. Exemplary materials include hydrophilic vinyl and acrylic polymers, polysaccharides such as calcium alginate, polyethylene oxide (PEO), poly-ethylene glycol (PEG), polypropylene glycol (PPG). Exemplary naturally occurring polymers include naturally occurring polysaccharides such as chitin, chitosan, dextran and pullulan; gum agar, gum arabic, gum karaya, locust bean gum, gum tragacanth, carrageenans, gum ghatti, guar gum, xanthan gum and scleroglucan; starches such as dextrin and maltodextrin; hydrophilic colloids such as pectin; phosphatides such as lecithin; alginates such as ammonium alginate, sodium, potassium or calcium alginate, propylene glycol alginate; collagen; and cellulosics, or combinations thereof. "Cellulosics" are understood das a cellulose polymer that has been modified by reaction of at least a portion of the hydroxyl groups on the saccharide repeat units with a compound to form an ester linked or an ether-linked substituent. For example, the cellulosic ethyl cellulose has an ether linked ethyl substituent attached to the saccharide repeat unit, while the cellulosic cellulose acetate has an ester linked acetate substituent.

A preferred class of cellulosics for the erodible matrix comprises aqueous-soluble and aqueous-erodible cellulosics such as ethyl cellulose (EC), methyl ethyl cellulose (MEC), carboxymethyl cellulose (CMC), carboxymethyl ethyl cellulose (CMEC), hydroxyethyl cellulose (HEC), hydroxypropyl cellulose (HPC), cellulose acetate (CA), cellulose propionate (CPr), cellulose butyrate (CB), cellulose acetate butyrate (CAB), cellulose acetate phthalate (CAP), cellulose acetate trimellitate (CAT), hydroxypropyl methyl cellulose (HPMC), hydroxypropyl methyl cellulose phthalate (HPMCP), hydroxypropyl methyl cellulose acetate succinate (HPMCAS), hydroxypropyl methyl cellulose acetate trimellitate (HPMCAT), and ethyl hydroxy ethyl cellulose (EHEC), or combinations thereof. A particularly preferred class of such cellulosics comprises various grades of low viscosity (MW less than or equal to 50,000 Daltons) and high viscosity (MW greater than 50,000 Daltons) HPMC. Commercially available low viscosity HPMC polymers include the Dow METHOCEL series E5, E15LV, E50LV and K100LY, while high viscosity HPMC polymers include E4MCR, E100MCR, K4M, K15M and K100M; especially preferred in this group are the METHOCEL™ K series. Other commercially available types of HPMC include the Shin Etsu METOLOSE 90SH series.

Further materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGIT®, Rohm America, Inc., Piscataway, N.J.) and other acrylic acid derivatives such as homopolymers and copolymers of butyl-methacrylate, methylmethacrylate, ethylmethacrylate, ethylacrylate, (2-dimethylaminoethyl)-methacrylate, and (trimethyl aminoethyl) methacrylate chloride, or combinations thereof.

The erodible matrix polymer may also contain a wide variety of additives and excipients known in the pharmaceutical arts, including osmopolymers, osmagens, solubility-enhancing or - retarding agents and excipients that promote stability or processing of the dosage form.

In alternative embodiments of SR formulation comprising the solubility-enhanced API, the sustained-release means may be a non-erodible matrix dosage form. In such dosage forms, the solubility-enhanced API is distributed in an inert matrix. The API is released by diffusion through the inert matrix. Examples of materials suitable for the inert matrix include insoluble plastics, such as copolymers of ethylene and vinyl acetate, methyl acrylate-methyl meth-acrylate copolymers, polyvinyl chloride, and polyethylene; hydrophilic polymers, such as ethyl cellulose, cellulose acetate, and crosslinked polyvinylpyrrolidone (also known as crospovidone); and fatty compounds, such as carnauba wax, microcrystalline wax, and triglycerides.

## Osmotic Sustained Release formulations

In a further alternative embodiment of the osmotic SR formulation forms may be used to incorporate the solubility-enhanced API. These dosage forms have at least two components: (a) the core which contains an osmotic agent and the solubility-enhanced API; and (b) a water permeable, non-dissolving and non-eroding coating surrounding the core. For these formulations the insoluble coating in aqueous media controls the influx of water to the core from an aqueous environment of use creating an osmotic pressure in the core. The non-eroding coating has at least one delivery port which is physically created during the manufacturing process. Because of the osmotic pressure some or all of the core, at least the part containing the API is extruded through the delivery port to the environment of use. The osmotic agent contained in the core of this dosage form may be an aqueous-swellable hydrophilic polymer or it may be an osmogen, also known as an osmagent. The coating is preferably polymeric, aqueous-permeable, and preformed or formed in situ. Examples of such dosage forms are well known in the art.

In addition to the solubility-enhanced API, the core of the osmotic dosage form optionally includes an "osmotic agent", i.e., any agent that creates a driving force for transport of water from the environment of use into the core of the dosage form. Exemplary osmotic agents are water-swellable hydrophilic polymers, and osmogens (or osmagens). Thus, the core may include water-swellable hydrophilic polymers, both ionic and non-ionic, commonly known as "osmopolymers" and "hydrogels". The amount of water-swellable hydrophilic polymers present in the core may range from about 5 to about 80 wt%, preferably 10 to 50 wt%. Exemplary materials include hydrophilic vinyl and acrylic polymers, polysaccharides such as calcium alginate, polyethylene oxide (PEO), poly-ethylene glycol (PEG), polypropylene glycol (PPG), poly(2-hydroxyethyl methacrylate), poly(acrylic) acid, poly-(methacrylic) acid, polyvinylpyrrolidone (PVP) and crosslinked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydrox-ypropyl methyl cellulose (HPMC), carboxymethyl cellulose (CMC) and carboxyethyl cellulose (CEC), sodium alginate, polycarbophil, xanthan gum, and sodium starch glycolate. Other materials include hydrogels comprising interpenetrating networks of polymers that may be formed by addition or by condensation polymerization, the components of which may comprise hydrophilic and hydrophobic monomers such as those just mentioned. Preferred polymers for use as the water-swellable hydrophilic polymers include PEO, PEG, PVP, sodium croscarmellose, HPMC, sodium starch glycolate, polyacrylic acid and crosslinked versions or mixtures thereof.

The core of the SR formulation according to the invention may further include an osmogen (or osmagent). The amount of osmogen present in the core may range from about 2 to about 70 wt%, preferably 10 to 50 wt%. Typical classes of suitable osmogens are water-soluble organic acids, salts and sugars that are capable of imbibing water to thereby effect an osmotic pressure gradient across the barrier of the surrounding coating. Typical useful osmogens include magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. Particularly preferred osmogens are glucose, lactose, sucrose, mannitol, xylitol and sodium chloride.

The core of the SR formulation of the invention may further include a wide variety of additives and excipients that enhance the performance of the dosage form or that promote stability, tableting or processing. Such additives and excipients include tableting aids, surfactants, water-soluble polymers, pH modifiers, fillers, binders, pigments, disintegrants, antioxidants, lubricants and flavorants. Exemplary of such components are microcrystalline cellulose; metallic salts of acids such as aluminium stearate, calcium stearate, magnesium stearate, sodium stearate, and zinc stearate; pH control agents such as buffers, organic acids and organic acid salts and organic and inorganic bases; fatty acids, hydrocarbons and fatty alcohols such as stearic acid, palmitic acid, liquid paraffin, stearyl alcohol, and palmitol; fatty acid esters such as glyceryl (mono- and di-) stearates, triglycerides, glyceryl (palmitic stearic) ester, sorbitan esters, such as sorbitan monostearate, saccharose monostearate, saccharose monopalmitate, and sodium stearyl fumarate; polyoxyethylene sorbitan esters; surfactants, such as alkyl sulfates such as sodium lauryl sulfate and magnesium lauryl sulfate; polymers such as polyethylene glycols, polyoxyethylene glycols, polyoxyethylene and polyoxypropylene ethers and their copolymers, and polytet-rafluoroethylene; and inorganic materials such as talc and dibasic calcium phosphate; cyclodextrins; sugars such as lactose and xylitol; and sodium starch glycolate. Examples of disintegrants are sodium starch glycolate (e.g., Explotab™), microcrystalline cellulose (e.g., Avicel™), microcrystalline silicified cellulose (e.g., ProSolv™), cros-carmellose sodium (e.g., Ac-Di-Sol™), or mixtures thereof.

For osmotic sustained release formulations, the rate of API release is controlled by such factors as the permeability and thickness of the coating, the osmotic pressure of the API-containing layer or API free layer, the degree of hydrophilicity of the hydrogel layer, and the dimension of the delivery port applied to the dosage form. Those skilled in the art will appreciate that reducing amount of osmotic active components in the core or reducing the size of the delivery port in the coating will reduce the release rate, while any of the following will increase the release rate: increasing the permeability of the coating; increasing the hydrophilicity of the hydrogel layer; increasing the osmotic pressure of the API containing or the API free layer; or increase the size of the delivery port.

Exemplary materials useful in forming the API-containing composition, in addition to solubility-enhanced API, include HPMC, PEO and PVP and other pharmaceutically acceptable carriers. In addition, osmagents such as sugars or salts, especially sucrose, lactose, xylitol, mannitol, or sodium chloride, may be added. Materials which are useful for forming the hydrogel layer include sodium CMC, PEO, poly (acrylic acid), sodium (polyacrylate), sodium croscarmellose, sodium starch glycolate, PVP, crosslinked PVP, and other high molecular weight hydrophilic materials or mixtures thereof. Particularly useful are PEO polymers having an average molecular weight from about 5,000,000 to about 7,500,000 Da.

As stated above the SR formulation according to the invention may have several distinct functional layers, apart from the API containing layer there may be a further sweller layer comprising a water-swellable polymer. In embodiments of the SR formulation, wherein the formulation comprises a bilayer geometry, the delivery port(s) or exit passageway(s) may be located on the side of the tablet containing the API composition or may be on both sides of the tablet or even on the edge of the tablet so as to connect both the API layer and the sweller layer with the exterior of the dosage form. The exit passageway(s) or delivery port may be produced by mechanical means or by laser drilling, or by creating a difficult-to-coat region on the tablet by use of special tooling during tablet compression or by other means.

An exemplary embodiment of an osmotic dosage form comprises: (a) a single-layer compressed core comprising: (i) solubility-enhanced API, (ii) a hydroxyethylcellulose, and (iii) an osmagent, wherein the hydroxyethylcellulose is present in the core from about 2.0% to about 35% by weight and the osmagent is present from about 15% to about 70% by weight; (b) a water-permeable and API-impermeable layer surrounding the core; and (c) at least one passageway within the layer (b) for delivering the API to a fluid environment surrounding the tablet. In a preferred embodiment, the dosage form is shaped such that the surface area to volume ratio (of a water-swollen tablet) is greater than 0.6 mm$^{-1}$; more preferably greater than 1.0 mm$^{-1}$. It is preferred that the passageway connecting the core with the fluid environment be situated along the tablet band area. A particularly preferred shape is an oblong shape where the ratio of the tablet tooling axes, i.e., the major and minor axes which define the shape of the tablet, are between 1.3 and 3; more preferably between 1.5 and 2.5. In one embodiment, the combination of solubility-enhanced API and the osmagent have an average ductility from about 100 to about 200 MPa, an average tensile strength from about 0.8 to about 2.0 MPa, and an average brittle fracture index less than about 0.2. The single-layer core may optionally include a disintegrant, a bioavailability enhancing additive, and/or a pharmaceutically acceptable excipient, carrier or diluent.

In further embodiments of the invention, the SR formulations may contain disintegrants that serve to break up the compressed core into its particulate components. Examples of standard disintegrants include materials such as sodium starch glycolate (e.g., Explotab™ CLV), microcrystalline cellulose (e.g., Avicel™), microcrystalline silicified cellulose (e.g., ProSolv™) and croscar-mellose sodium (e.g., Ac-Di-Sol™), and other disintegrants known to those skilled in the art. Depending upon the particular formulation, some disintegrants work better than others. Several disintegrants tend to form gels as they swell with water, thus hindering drug delivery from the dosage form. Non-gelling, non-swelling disintegrants provide a more rapid dispersion of the solubility enhanced API complex particles within the core as water enters the core. Preferred non-gelling, non-swelling disintegrants are resins, preferably ion-exchange resins. A preferred resin is Amberlite™ IRP 88 (available from Rohm and Haas, Philadelphia, Pa.). When used, the disintegrant is present in amounts ranging from about 1-25% of the core composition.

Water-soluble polymers are added to keep particles of the solubility-enhanced API suspended inside the SR formulation before they can be delivered through the passageway(s) (e.g., an orifice). High viscosity polymers are useful in preventing settling. However, the polymer in combination with the API is extruded through the passageway(s) under relatively low pressures. At a given extrusion pressure, the extrusion rate typically slows with increased viscosity. Certain polymers in combination with particles of the drug form high viscosity solutions with water but are still capable of being extruded from the tablets with a relatively low force. In contrast, polymers having a low weight-average, molecular weight (< about 300,000) do not form sufficiently viscous solutions inside the tablet core to allow complete delivery due to particle settling. Settling of the particles is a problem when such dosage forms are prepared with no polymer added, which leads to poor API delivery.

Preferred water-soluble polymers for such osmotic dosage forms do not interact with the API. Non-ionic polymers are preferred. An example of a non-ionic polymer forming solutions having a high viscosity yet still extrudable at low pressures is Natrosol™ 250H (high molecular weight hydroxyethylcellulose, available from Hercules Incorporated, Aqualon Division, Wilmington, Del.; MW equal to about 1,000,000 Da and a degree of polymerization equal to about 3,700). Natrosol™ 250H provides effective drug delivery at concentrations as low as about 3% by weight of the core when combined with an osmagent. Natrosol™ 250H NF is a high-viscosity grade non-ionic cellulose ether that is soluble in hot or cold water. The viscosity of a 1 % solution of Natrosol™ 250H using a Brookfield LVT (30 rpm) at 25° C is between about 1,500 and about 2,500 cps.

Preferred hydroxyethylcellulose polymers for use in these monolayer osmotic tablets have a weight-average, molecular weight from about 300,000 to about 1.5 million. The hydroxyethylcellulose polymer is typically present in the core in an amount from about 2.0% to about 35% by weight.

Another class of SR formulations useful in this invention comprises coated swellable tablets. Coated swellable tablets comprise a tablet core comprising the solubility-enhanced API of the drug and a swelling material, preferably a hydrophilic polymer, coated with a membrane, which contains holes, or pores through which, in the aqueous use environment, the swelling hydrophilic polymer can extrude and carry out the API composition. Alternatively, the membrane may contain polymeric or low molecular weight water-soluble "porosigens". Porosigens dissolve in the aqueous use environment, providing holes or pores through which the hydrophilic polymer and drug may extrude or be released by diffusion or both. Examples of porosigens are water-soluble polymers such as HPMC, PEG, and low molecular weight compounds such as glycerol, sucrose, glucose, and sodium chloride. In addition, pores may be formed in the coating by drilling holes in the coating using a laser, mechanical, or other means. In this class of osmotic dosage forms, the membrane material may comprise any film-forming polymer, including polymers which are water permeable or impermeable, providing that the membrane deposited on the tablet core is porous or contains water-soluble porosigens or possesses a macroscopic hole for water ingress and API release.

A preferred form of coating is a semipermeable polymeric membrane that has the port(s) formed therein either prior to or during use. Thickness of such a polymeric membrane may vary between about 20 and 800 μm and is preferably in the range of 100 to 500 μm. The delivery port(s) should generally range in size from 0.1 to 3000 μm or greater, preferably on the order of 50 to 3000 μm in diameter. Such port(s) may be formed post-coating by mechanical or laser drilling or may be formed in situ by rupture of the coatings; such rupture may be controlled by intentionally incorporating a relatively small weak portion into the coating. Delivery ports may also be formed in situ by erosion of a plug of water-soluble material or by rupture of a thinner portion of the coating over an indentation in the core. In addition, delivery ports may be formed during coating, as in the case of asymmetric membrane coatings.

Coatings may be dense, microporous or "asymmetric", having a dense region supported by a thick porous region. When the coating is dense the coating is composed of a water-permeable material. When the coating is porous, it may be composed of either a water-permeable or a water-impermeable material. When the coating is composed of a porous water-impermeable material, water permeates through the pores of the coating as either a liquid or a vapor.

Materials useful in forming the coating include various grades of acrylics, vinyls, ethers, polyamides, polyesters and cellulosic derivatives that are water-permeable and water-insoluble at physiologically relevant pHs, or are susceptible to being rendered water-insoluble by chemical alteration such as by crosslinking.

Specific examples of suitable polymers (or crosslinked versions) useful in forming the coating include plasticized, unplasticized and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethy-laminoacetate, CA ethyl carbonate, CA chloroacetate, CA ethyl oxalate, CA methyl sulfonate, CA butyl sulfonate, CA p-toluene sulfonate, agar acetate, amylase triacetate, beta glucan acetate, beta glucan triacetate, acetaldehyde dimethyl acetate, triacetate of locust bean gum, hydroxlated ethylene-vinylacetate, and ethyl cellulose, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) acids and esters and copolymers thereof, starch, dextran, dextrin, chitosan, collagen, polyalkenes, polyethers, polysulfones, polyethersulfones, polystyrenes, polyvinyl halides, polyvinyl esters and ethers, natural waxes and synthetic waxes.

A preferred coating composition comprises a cellulosic polymer, in particular cellulose ethers, cellulose esters and cellulose ester-ethers, i.e., cellulosic derivatives having a mixture of ester and ether substituents. Another preferred class of coating materials are poly(acrylic) acids and esters, poly(methacrylic) acids and esters, and copolymers thereof. A more preferred coating composition comprises cellulose acetate. An even more preferred coating comprises a cellulosic polymer and PEG. A most preferred coating comprises cellulose acetate and PEG.

Coating is performed by conventional means and techniques, usually by dissolving or suspending the coating material in a solvent and then coating by dipping, pan-coating or preferable spray coating or most preferably fluid bed spray coating by pan-coating. A preferred coating solution contains 1 to 10 wt% polymer, preferably 2 to 8 wt% polymer, most preferably 3 to 8 wt% polymer, preferably about 4 wt% polymer. Typical solvents useful with the cellulosic polymers mentioned above include acetone, methyl acetate, ethyl acetate, isopropyl acetate, n-butyl acetate, methyl isobutyl ketone, methyl propyl ketone, ethylene glycol monoethyl ether, ethylene glycol monoethyl acetate, methylene dichloride, ethylene dichloride, propylene dichloride, nitroethane, nitropropane, tetrachloroethane, 1,4-dioxane, tetrahydrofuran, diglyme, water, and mixtures thereof. Pore-formers and non-solvents (such as water, glycerol and ethanol) or plasticizers (such as diethyl phthalate) may also be added in any amount as long as the polymer remains soluble at the spray temperature.

Coatings may also be hydrophobic microporous layers wherein the pores are substantially filled with a gas and are not wetted by the aqueous medium but are permeable to watervapor. Such hydrophobic but water-vapor permeable coatings are typically composed of hydrophobic polymers such as polyalkenes, polyacrylic acid derivatives, polyethers, polysulfones, polyethersulfones, polystyrenes, polyvinyl halides, polyvinyl esters and ethers, natural waxes and synthetic waxes. Especially preferred hydrophobic microporous coating materials include polystyrene, polysulfones, polyethersulfones, polyethylene, polypropylene, polyvinyl chloride, polyvinylidene fluoride and polytetrafluoroethylene. Such hydrophobic coatings can be made by known phase inversion methods using any of vapor-quench, liquid quench, thermal processes, leaching soluble material from the coating or by sintering coating particles. In thermal processes, a solution of polymer in a latent solvent is brought to liquid-liquid phase separation in a cooling step. When evaporation of the solvent is not prevented, the resulting membrane will typically be porous.

Diffusion membrane Sustained Release formulations

In a further alternative embodiment of the SR formulation according to this invention diffusion membrane sustained release dosage form may be used to incorporate the solubility-enhanced API. These dosage forms have at least two components: (a) the tablet core containing one or more pharmaceutical acceptable excipients used for formulation of conventional tablets and the solubility-enhanced API; and (b) a water permeable, aqueous non-dissolving and non-eroding coating surrounding of the tablet core containing at least a (i) water insoluble membrane forming polymer (ii) a swellable pore forming hydrophilic polymer, (iii) a hydrophilic or hydrophobic plasticizer and other water-soluble excipients or leachable hydrophobic excipients. In these formulations the hydrophilic and leachable components are forming pores in the membrane in the aqueous environment of use. The solubility enhanced API-complex is released to the environment of use by diffusion through this in-situ created diffusion membrane. The coating may contain other excipients optimizing the coating process or the performance of the diffusion membrane.

For diffusion membrane sustained release formulations the rate of API release is controlled by such factors as the permeability provided by the hydrophilic components included in the coated membrane and thickness of the coating provided, the surface area of the dosage tablet. Those skilled in the art will appreciate that reducing the amount of hydrophilic components (pore builders) in the coated membrane and thus reducing its permeability, increasing the thickness of the membrane and reduction of the surface area of the dosage form will reduce the release rate: increasing the amount of pore building excipients in the coated membrane and thus increasing its permeability, reducing the thickness of the membrane and increasing the surface area of the dosage form will increase the release rate of the solubility enhanced API.

Examples of such dosage forms are well known in the art.

In addition to the solubility-enhanced API, the tablet core of the diffusion membrane dosage form optionally may include swellable hydrophilic and hydrophobic polymers to enhance creation of the pores. However, the amount of those polymers present in the tablet core is limited to that extend that the diffusion membrane shall remain intact during administration to avoid immediate release of the solubility enhanced API because of uncontrolled ruptures.

The amount may range from about 1 to about 20 wt%, preferably 1 to 10 wt%. Exemplary materials include hydrophilic vinyl and acrylic polymers, polysaccharides such as calcium alginate, polyethylene oxide (PEO), poly-ethylene glycol (PEG), polypropylene glycol (PPG), poly(2-hydroxyethyl methacrylate), poly(acrylic) acid, poly-(methacrylic) acid, polyvinylpyrrolidone (PVP) and crosslinked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydrox-ypropyl methyl cellulose (HPMC), carboxymethyl cellulose (CMC) and carboxyethyl cellulose (CEC), sodium alginate, polycarbophil, xanthan gum, and sodium starch glycolate. Other materials include hydrogels comprising interpenetrating networks of polymers that may be formed by addition or by condensation polymerization, the components of which may comprise hydrophilic and hydrophobic monomers such as those just mentioned. Preferred polymers for use as the water-swellable hydrophilic polymers include PEO, PEG, PVP, sodium croscarmellose, HPMC, sodium starch glycolate, polyacrylic acid and crosslinked versions or mixtures thereof.

The Tablet core of the SR formulation of the invention may further include a wide variety of additives and excipients that enhance the performance of the dosage form or that promote stability, tableting or processing. Such additives and excipients include tableting aids, surfactants, water-soluble polymers, pH modifiers, fillers, binders, pigments, disintegrants, antioxidants, lubricants and flavorants. Exemplary of such components are microcrystalline cellulose; metallic salts of acids such as aluminium stearate, calcium stearate, magnesium stearate, sodium stearate, and zinc stearate; pH control agents such as buffers, organic acids and organic acid salts and organic and inorganic bases; fatty acids, hydrocarbons and fatty alcohols such as stearic acid, palmitic acid, liquid paraffin, stearyl alcohol, and palmitol; fatty acid esters such as glyceryl (mono- and di-) stearates, triglycerides, glyceryl (palmitic stearic) ester, sorbitan esters, such as sorbitan monostearate, saccharose monostearate, saccharose monopalmitate, and sodium stearyl fumarate; polyoxyethylene sorbitan esters; surfactants, such as alkyl sulfates such as sodium lauryl sulfate and magnesium lauryl sulfate; polymers such as polyethylene glycols, polyoxyethylene glycols, polyoxyethylene and polyoxypropylene ethers and their copolymers, and polytet-rafluoroethylene; and inorganic materials such as talc and dibasic calcium phosphate; cyclodextrins; sugars such as lactose and xylitol; and sodium starch glycolate. Examples of disintegrants are sodium starch glycolate (e.g., Explotab™), microcrystalline cellulose (e.g., Avicel™), microcrystalline silicified cellulose (e.g., ProSolv™), cros-carmellose sodium (e.g., Ac-Di-Sol™), or mixtures thereof.

Membrane forming polymers used for diffusion membrane sustained release formulation are selected from cellulosic polymer, in particular cellulose ethers, cellulose esters and cellulose ester-ethers, i.e., cellulosic derivatives having a mixture of ester and ether substituents. Another class of coating materials are poly(acrylic) acids and esters, poly(methacrylic) acids and esters, and copolymers thereof. The most preferred diffusion membrane forming polymer is ethyl-cellulose in various viscosity and substitution grades.

Hydrophilic polymers used as pore forming excipients incorporated in the diffusion membrane may generally be described as an osmopolymer, hydrogel or water-swellable polymer. Such polymers may be linear, branched, or crosslinked. They may be homopolymers or copolymers. Although they may be synthetic polymers derived from vinyl, acrylate, methacrylate, urethane, ester and oxide monomers, they are most preferably derivatives of naturally occurring polymers such as polysaccharides or proteins. Exemplary materials include hydrophilic vinyl and acrylic polymers, polysaccharides such as calcium alginate, polyethylene oxide (PEO). Exemplary naturally occurring polymers include naturally occurring polysaccharides such as chitin, chitosan, dextran and pullulan; gum agar, gum arabic, gum karaya, locust bean gum, gum tragacanth, carrageenans, gum ghatti, guar gum, xanthan gum and scleroglucan; starches such as dextrin and maltodextrin; hydrophilic colloids such as pectin; phosphatides such as lecithin; alginates such as ammonium alginate, sodium, potassium or calcium alginate, propylene glycol alginate; collagen; and cellulosics, or combinations thereof. "Cellulosics" are understood as a cellulose polymer that has been modified by reaction of at least a portion of the hydroxyl groups on the saccharide repeat units with a compound to form an ester linked or an ether-linked substituent. For example, the cellulosic ethyl cellulose has an ether linked ethyl substituent attached to the saccharide repeat unit, while the cellulosic cellulose acetate has an ester linked acetate substituent. Most preferred polymeric pore builders are hydroxyproplycellulose, Hydroxypropyl methyl cellulose and polyvinylpyrolidone in various grades and viscosity. Preferred water-soluble polymers for such diffusion membrane dosage forms do not interact with the API. Non-ionic polymers and fillers are preferred.

The coating may include conventional plasticizers, including glycerine, propylene glycol, dibutyl phthalate; dibutyl sebacate; diethyl phthalate; dimethyl phthalate; triethyl citrate; benzyl benzoate; butyl and glycol esters of fatty acids; mineral oil; oleic acid; stearic acid; cetyl alcohol; stearyl alcohol; castor oil; corn oil; coconut oil; and camphor oil; and other excipients such as anti-tack agents, glidants, etc. For plasticizers, triethyl citrate, propylene glycol and dibutyl sebacate are particularly preferred. Typically, the coating may include from about 0.1 to about 25 wt.% plasticizer and from about 0.1 to about 10 wt % of other excipients like anti-tack agents or colorants.

Coating is performed by conventional means and techniques, usually by dissolving or suspending the coating material in a solvent and then coating by dipping, spray coating or by pan-coating. A preferred coating solution contains 1 to 8 wt% polymer, preferably 2 to 6 wt% polymer, preferably about 4 to 5 wt% polymer. Solvents may be used to apply the coating to the tablet cores containing the solubility enhanced API. Typical solvents suitable for use in applying coating to a diffusion membrane coated sustained release tablet core include alcohols, such as methanol, ethanol, isomers of propanol and isomers of butanol; ketones, such as acetone, methyl ethyl ketone and methyl isobutyl ketone; hydrocarbons, such as pentane, hexane, heptane, cyclohexane, methylcyclohexane, octane and mineral oil; ethers, such as methyl tert-butyl ether, ethyl ether and ethylene glycol monoethyl ether; chlorocarbons, such as chloroform, methylene dichloride and ethylene dichloride; tetrahydrofuran; dimethyl sulfoxide; N-methyl pyrrolidinone; acetonitrile; water; and mixtures thereof. Most preferred solvents are methanol, Isopropylic alcohol, ethanol and mixtures thereof with water. Pore-formers and non-solvents or plasticizers may also be added in any amount as long as the polymer remains soluble at the spray temperature.

Coatings may also be hydrophobic microporous layers wherein the pores are substantially filled with a gas and are not wetted by the aqueous medium but are permeable to water vapor. Such hydrophobic but water-vapor permeable coatings are typically composed of hydrophobic polymers such as polyalkenes, polyacrylic acid derivatives, polyethers, polysulfones, polyethersulfones, polystyrenes, polyvinyl halides, polyvinyl esters and ethers, natural waxes and synthetic waxes. Especially preferred hydrophobic microporous coating materials include polystyrene, polysulfones, polyethersulfones, polyethylene, polypropylene, polyvinyl chloride, polyvinylidene fluoride and polytetrafluoroethylene. Such hydrophobic coatings can be made by known phase inversion methods using any of vapor-quench, liquid quench, thermal processes, leaching soluble material from the coating or by sintering coating particles. In thermal processes, a solution of polymer in a latent solvent is brought to liquid-liquid phase separation in a cooling step. When evaporation of the solvent is not prevented, the resulting membrane will typically be porous.

To achieve best results, one or more polymeric or non-polymeric release retardants may be selected from the group comprising cellulosic polymers, cellulose derivatives, sodium carboxymethylcellulose, hydroxyalkyl celluloses such as hydroxypropyl methylcellulose and hydroxypropyl cellulose, polyalkylene oxides, polyethylene oxide, alkyl celluloses such as methylcellulose and ethyl cellulose, hydroxypropyl methyl cellulose, hydroxyethyl cellulose, hydroxypropyl cellulose, polyethylene glycol, cellulose acetate, cellulose acetate butyrate, ammonio methacrylate copolymer, polyacrylate dispersion, polyvinylpyrrolidone polyvinyl acetate, acrylic acid, methacrylic acid polymers, and esters thereof, maleic anhydride polymers, polymaleic acid, poly(acrylamides), poly(olefinic alcohols, poly(N-vinyl lactams), polyols, polyoxyethylated saccharides, polyoxazolines, polyvinyl amines, polyvinyl acetates, polyimines, starch and starch-based polymers, polyurethane hydrogels, chitosan, polysaccharide gums, zein, shellac-based polymers, polyethylene oxide, sodium carboxy methylcellulose, calcium carboxymethyl cellulose, methyl cellulose, polyacrylic acid, maltodextrin, pregelatinized starch and polyvinyl alcohol, copolymers, and mixtures thereof.

One or more hydrophilic polymers may also be used to prepare sustained release dosage forms. These polymers are preferably polyethylene oxide, hydroxypropyl cellulose, hydroxypropyl methyl cellulose, hydroxyethyl cellulose, sodium carboxy methylcellulose, calcium carboxymethyl cellulose, methyl cellulose, polyacrylic acid, maltodextrin, pregelatinized starch, polyvinyl alcohol, or mixtures thereof. The weight percent of the hydrophilic polymer in the SR formulation is about 5 to about 90 wt%, preferably about 10-80 wt%, preferably about 10-70 wt%, preferably about 10-60 wt%, preferably about 10-50 wt%, more preferably about 15 -50 wt%, more preferably about 25 -50 wt% and most preferably about 30-50 wt%.

Most preferred is that the retard polymer is ethyl cellulose.

Examples of solvents suitable for use in applying a coating to an enteric coated sustained release core include alcohols, such as methanol, ethanol, isomers of propanol and isomers of butanol; ketones, such as acetone, methyl ethyl ketone and methyl isobutyl ketone; hydrocarbons, such as pentane, hexane, heptane, cyclohexane, methylcyclohexane, octane and mineral oil; ethers, such as methyl tert-butyl ether, ethyl ether and ethylene glycol monoethyl ether; chlorocarbons, such as chloroform, methylene dichloride and ethylene dichloride; tetrahydrofuran; dimethyl sulfoxide; N-methyl pyrrolidinone; acetonitrile; water; and mixtures thereof.

Multi-particulate Sustained Release formulations

In further alternative embodiment the SR formulation according to the invention may be a multi-particulate formulation. Multi-particulates generally refer to dosage forms that comprise a multiplicity of particles or granules that may range in size from about 10 $\mu$m to about 3 mm, more typically about 50 $\mu$m to 2 mm in diameter. A therapeutic effective dose of the API is provided by a multiple of the individual particulates. Such multi particulates may be packaged, for example, in a capsule such as a gelatine capsule or a capsule formed from an aqueous-soluble polymer such as HPMCAS, HPMC or starch; dosed as a suspension or slurry in a liquid; or they may be formed into a tablet, caplet, or pill by compression or other processes known in the art.

Such multi-particulates may be made by any known process, such as wet- and dry-granulation processes, extrusion/spheronization, tablet compression, roller-compaction, melt-congealing, or by spray-coating seed cores. For example, in wet- and dry-granulation processes, the composition comprising the solubility-enhanced API according to the invention and optional excipients may be granulated to form multi-particulates of the desired size. Other excipients, such as a binder (e.g., microcrystalline cellulose), may be blended with the composition to aid in processing and forming the multi-particulates. In the case of wet granulation, a binder such as microcrystalline cellulose may be included in the granulation fluid to aid in forming a suitable multi-particulate.

Multi particulate sustained release formulations may comprise a plurality of diffusion membrane sustained release formulations with one or more API of which at least one contains a solubility enhanced API as described above.

In any case, the resulting particles may themselves constitute the multi-particulate dosage form or they may be coated by various film-forming materials such as diffusion membrane forming excipients and polymers, gastric resistant enteric polymers or water-swellable or water-soluble polymers, or they may be combined with other excipients or vehicles to aid in dosing to patients.

Multi-particulate embodiments of the SR formulation of the invention have the further advantage that a blend of different release retarding system can be used, such as sustained-release pellets coated with a diffusion membrane and a sustained-release matrix particulate. This allows easily and independently to vary the release kinetics for each active substance, the proportion of the active substances and combining different APIs, for tailoring the treatment to a patient.

Multi-particulates may also comprise more than one solubility enhanced API complex in different types of particulates which allow for combination therapies.

Multi-layered Sustained Release formulations

For some embodiments of the SR formulation according to the invention the formulation may comprise multilayer tablets. As with multi-particulates multi-layered SR formulations may comprise more than one solubility enhanced API complex for parallel treatment of related or associated diseases or symptoms, wherein variable release kinetics for each API are easily achieved and individualised treatments can therefore easily be optimised.

Multilayer tablet embodiments of the SR formulation of the invention have the further advantage that in each layer a different release retarding system can be used, such as sustained-release pellets coated with a release retardant, or a sustained-release matrix. This allows easily and independently to vary the release kinetics for each solubility enhanced API complex and the proportion of the solubility enhanced API complexes, for tailoring the treatment to a patient. Alternatively, a single solubility enhanced API complex may be provided in distinct release stages with different release profiles which are selected from the group comprising immediate release, sustained/retarded/prolonged release, extended release, controlled release or delayed release.

The multilayer tablet of the invention also has the advantage that it enables a much more accurate dosage of the API. This is particularly important for small doses.

The multilayer tablet of the invention is also advantageous when the two APIs are not compatible with one another.

According to a particularly preferred embodiment of the SR formulation of the invention, the multilayer tablet is a two-layer tablet containing two distinct APIs, wherein the formulation according to the invention comprises at least one solubility-enhanced low solubility API in the meaning of the present invention. The second present API may or may not be a low solubility API. If the second API is a low solubility API according to the present invention, the second API is also provided as a solubility enhanced API according to the invention. The multilayer tablet of the invention can be obtained as a two-layer tablet by a simple and therefore inexpensive process.

In a further embodiment of the multilayer tablet SR formulation according to the invention, the second API-containing layer comprises a matrix, which prolongs the release of the second (possibly solubility enhanced) API, or a pharmaceutically acceptable salt thereof. A release-prolonging matrix can be obtained by a simple and therefore inexpensive process. The matrix can, according to the invention, preferably be a so-called scaffold matrix, which can be swelling or non-swelling, or a so-called eroding matrix. The matrix can also have properties of both scaffold and eroding matrixes.

In a scaffold matrix, the API is incorporated into the matrix structure. The API is gradually dissolved by the digestive juices from the loaded scaffold matrix during the transport through the gastrointestinal tract. When this is done, the matrix scaffold is excreted in unchanged form, or in a swollen form. In contrast, when an eroding matrix is used, the matrix is degraded, or eroded, which leads to API particles being exposed at the surface and dissolved. The release rate therefore depends on the matrix degradation or erosion rate.

According to a further preferred embodiment of the multilayer tablet SR formulation of the invention, the matrix contains one or several water-insoluble matrix-forming agents. In an alternative embodiment, the matrix contains one or several water-soluble matrix-forming agents.

In another preferred embodiment of the multi-layer table SR formulation of the invention, the matrix of the composition comprises one or several matrix-forming agents selected from the group consisting of cellulose esters, polyethylene oxide, polyvinylpyrrolidone/polyvinyl acetate mixtures, methacrylate-acrylate copolymers, waxes, fats such as glycerol esters, and fatty alcohols. The substance classes mentioned here are particularly suitable as matrix-forming agents for the composition of the invention. However, particularly preferred is the use of a mixture of polyvinyl acetate and polyvinylpyrrolidone, and/ or a glycerol dibehenic acid ester as matrix-forming agent.

The release rate of any of the above SR formulations (multilayer SR, multi-particulate SR, diffusion membrane SR, matrix SR) is, in accordance with the invention, controlled as described above by the amount of hydrophilic components (pore builders), the thickness of the membrane and the surface area of the dosage, the permeability of the formulation and the matrix degradation or erosion rate.

In a further embodiment of the SR formulation, the multilayer tablet additionally comprises at least one stabilizer, which protects the matrix. In a preferred embodiment, the at least one stabilizer is selected from the list comprising butylated hydroxytoluol, sulphur dioxide, sodium sulphite, sodium bisulphite, ascorbic acid and its derivatives and tocopherol, as well as its water- and fat-soluble derivatives, such as, for example, tocopherol acetate, sulphites, bisulphites and hydrogen sulphites of alkali, alkaline earth metals and other metals, paraben, BHA, BHT, gallates as well as lower fatty acids, fruit acids, phosphoric acids, sorbic and benzoic acids and their salts, esters, derivatives and isomeric compounds, ascorbyl palmitate, lecithins, mono- and polyhydroxylated benzene derivatives, ethylenediaminetetraacetic acid and their salts, citraconic acid, cysteine, L-cysteine, conidendrin, diethyl carbonate, methylenedioxyphenole, cephalin, $\beta,\beta$'-dithiopropionic acid, biphenyl and other phenyl derivatives.

In a further embodiment, the multilayer tablet comprises a dye. In a preferred embodiment, the dye is selected from a group comprising red iron oxide, black iron oxide and indigo carmine.

Enteric coatings of Sustained Release formulations

In some embodiments of the above-described embodiments of SR formulation according to the invention, the sustained release means may comprise a core containing the solubility enhanced API complex coated with an enteric coating so that the core does not dissolve in the stomach. The core may be either a sustained release core, such as a SR matrix tablet or a SR diffusion membrane tablet or particle, or alternatively may be an immediate release core that provides a delayed burst or may be a multi-particulate or multi-layered core. "Enteric coating" is understood as an acid resistant coating that remains intact and does not dissolve at pH of less than about 4. The enteric coating surrounds the core so that the core does not dissolve in the stomach. The enteric coating may include an enteric coating polymer. Enteric coating polymers are generally polyacids having a pKa of about 3 to 5. Examples of enteric coating polymers include: cellulose derivatives, such as cellulose acetate phthalate, cellulose acetate trimellitate, hydroxypropyl methyl cellulose acetate succinate, cellulose acetate succinate, carboxy methyl ethyl cellulose, methylcellulose phthalate, and ethyl hydroxy cellulose phthalate; vinyl polymers, such as polyvinyl acetate phthalate, polyvinyl butyrate acetate, vinyl acetate-maleic anhydride copolymer; polyacrylates; and polymethacrylates such as methyl acrylate methacrylic acid copolymer, methacrylate-methacrylic acid octyl acrylate copolymer; and styrene-maleic mono-ester copolymer. These may be used either alone or in combination, or together with other polymers than those mentioned above.

The coating may include conventional plasticizers, including dibutyl phthalate; dibutyl sebacate; diethyl phthalate; dimethyl phthalate; triethyl citrate; benzyl benzoate; butyl and glycol esters of fatty acids; mineral oil; oleic acid; stearic acid; cetyl alcohol; stearyl alcohol; castor oil; corn oil; coconut oil; and camphor oil; and other excipients such as anti-tack agents, glidants, etc. For plasticizers, triethyl citrate, coconut oil and dibutyl sebacate are particularly preferred. Typically, the coating may include from about 0.1 to about 25 wt.% plasticizer and from about 0.1 to about 10 wt % anti-tack agents.

The enteric coating may be applied to the core by dissolving or suspending the enteric coating materials in a suitable solvent. Examples of solvents suitable for use in applying a coating include alcohols, such as methanol, ethanol, isomers of propanol and isomers of butanol; ketones, such as acetone, methyl ethyl ketone and methyl isobutyl ketone; hydrocarbons, such as pentane, hexane, heptane, cyclohexane, methylcyclohexane, and octane; ethers, such as methyl tert-butyl ether, ethyl ether and ethylene glycol monoethyl ether; chlorocarbons, such as chloroform, methylene dichloride and ethylene dichloride; tetrahydrofuran; dimethyl sulfoxide; N-methyl pyrrolidinone; acetonitrile; water; and mixtures thereof.

In this embodiment of the invention the sustained release formulation of the invention with an enteric coating as described previously, the enteric coating is applied below the sustained release layer. Hence, the API in the formulation of the present invention is first coated with an enteric coating, when producing SR pellets. This enteric coating is then followed by coating with a retard polymer producing a sustained release layer. This produces a tablet with a much more precise defined release starting point. The reason for this is that the sustained release coating becomes permeable in a time-dependent manner. This means that even if the point with a certain pH is attained early, the enteric coating that is inside the sustained release coating would not be affected until the time when the sustained release coating is sufficiently permeable for the enteric coating to be dissolved. The tablet is therefore able to mitigate the variability of the start of the release caused by the pH variability in the gut.

In the present dosage form, the enteric layer is coated with a sustained release (SR) layer, which ensures the controlled release and less variability from patient to patient in the delayed release phase as detailed above. The additional SR layer serves as additional diffusion membrane.

Enteric coated SR formulations may use release retardants as described above for diffusion membrane SR formulations.

Further excipients of Sustained Release formulations

For all above-described SR formulations of the invention the dosage forms may be inlayed with one or more pharmaceutically acceptable excipients, which are preferably not enteric coating polymers and are even more preferably pH-independent pharmaceutically acceptable excipients.

In further embodiments of the SR formulation according to the invention, the formulation may contain other excipients to improve performance, handling, or processing. Generally, excipients such as surfactants, pH modifiers, fillers, matrix materials, complexing agents, solubilizers, pigments, lubricants, glidants, flavorants, binder, plasticizer, pore builder, solvent and so forth may be used for customary purposes and in typical amounts without adversely affecting the properties of the sustained release dosage form.

Further, the SR formulation may also additionally comprise compounds for abuse prevention. Such compounds may be selected from the group of compounds comprising viscosity increasing agents, emetic, irritative substances, and/or antagonists of the active ingredient.

As noted, before, the present SR formulation may be used to administer a broad spectrum of APIs for a wide selection of diseases, symptoms and treatments due to its defined controlled release with little variability and improved safety. For example, the dosage form may be used, in the treatment of Attention Deficit Hyperactivity Disorder (ADHD), diabetes, pain, insomnia, high blood pressure, allergies such as allergic rhinitis, gastroesophageal reflux diseases, and many others.

In a further embodiment, the SR formulation additionally comprises at least one stabilizer, which protects the matrix. In a preferred embodiment, the at least one stabilizer is selected from the list comprising butylated hydroxytoluol, sulphur dioxide, sodium sulphite, sodium bisulphite, ascorbic acid and its derivatives and tocopherol, as well as its water- and fat-soluble derivatives, such as, for example, tocopherol acetate, sulphites, bisulphites and hydrogen sulphites of alkali, alkaline earth metals and other metals, paraben, BHA, BHT, gallates as well as lower fatty acids, fruit acids, phosphoric acids, sorbic and benzoic acids and their salts, esters, derivatives and isomeric compounds, ascorbyl palmitate, lecithins, mono- and polyhydroxylated benzene derivatives, ethylenediaminetetraacetic acid and their salts, citraconic acid, cysteine, L-cysteine, conidendrin, diethyl carbonate, methylenedioxyphenole, cephalin, $\beta,\beta'$-dithiopropionic acid, biphenyl and other phenyl derivatives.

In a preferred embodiment, the SR formulation comprises at least one additive, wherein this additive is an emetic. In a preferred embodiment, the emetic is based on one or several substances from radix ipecacuanha (ipecac). In a preferred embodiment, the emetic is based on the substance emetine, in an alternative embodiment, the emetic is apomorphine.

In a further embodiment, the SR formulation comprises a dye. In a preferred embodiment, the dye is selected from a group comprising red iron oxide, black iron oxide and indigo carmine.

In a further embodiment, the SR formulation may depend on the API additionally comprises at least one non-steroid antirheumatic or an antihistamine or other functionally similar API which is selected to counteract or mitigate possible side-effects of the API if medically and pharmaceutically required or recommended.

In an alternative embodiment, the SR formulation additionally comprises at least one water-soluble lubricant. In a preferred embodiment, the composition comprises at least one water-soluble lubricant selected from the group comprising adipic acid, fumaric acid, sodium benzoate and macrogol.

In some embodiments of the SR formulation of the invention, the composition may comprise surfactants. Preferably surfactants are present between 0 and 10 wt% of the SR formulation. Surfactant may be selected from, but are not limited to, the following exemplary surfactants which include fatty acid and alkyl sulfonates; commercial surfactants such as benzalkonium chloride (HYAMINE® 1622, available from Lonza, Inc., Fairlawn, N.J.); dioctyl sodium sulfosuccinate (DOCU-SATE SODIUM, available from Mallinckrodt Spec. Chem., St. Louis, Mo.); polyoxyethylene sorbitan fatty acid esters (TWEEN®, available from ICI Americas Inc., Wilmington, Del.; LIPOSORB® 0-20, available from Lipochem Inc., Patterson N.J.; CAPMUL® POE-0, available from Abitec Corp., Janesville, Wis.); and natural surfactants such as sodium taurocholic acid, I-palmitoyl-2-oleoyl-sn-glycero-3-phosphocholine, lecithin, and other phospholipids and mono- and diglycerides. Such materials can advantageously be employed to increase the rate of dissolution by, for example, facilitating wetting, or other-wise increase the rate of drug release from the dosage form.

In some embodiments of the invention the SR formulation may further comprise additional solubilizing agents. One preferred class of solubilizing agents consists of organic acids. Solubilizing agents may be selected from, but are not limited to, the following exemplary organic acids which include acetic, aconitic, adipic, ascorbic, aspartic, benzenesulfonic, benzoic, camphor sulfonic, cholic, citric, decanoic, erythorbic, 1,2-ethanedisulfonic, ethane sulfonic, formic, fumaric, gluconic, glucuronic, glutamic, glutaric, glyoxylic, heptanoic, hippuric, hydroxyethanesulfonic, lactic, lactobionic, levulinic, lysine, maleic, malic, malonic, mandelic, methane sulfonic, mucic, 1- and 2-naphthalenesulfonic, nicotinic, pamoic, pantothenic, phenylalanine, 3-phenylpropionic, phthalic, salicylic, saccharic, succinic, tannic, tartaric, p-toluene sulfonic, tryptophan, and uric.

Solubilizing agents may further comprise lipophilic microphase-forming materials. Lipophilic microphase-forming material may comprise a surfactant and/or a lipophilic material. Some amphiphilic materials may not be water immiscible by themselves, but instead are at least somewhat water soluble. Such amphiphilic materials may nevertheless be used in mixtures to form the lipophilic microphase, particularly when used as mixtures with hydro-phobic materials. Thus, as used herein, the "lipophilic microphase-forming material" is intended to include blends of materials in addition to a single material. Examples of amphiphilic materials suitable for use as the lipophilic microphase-forming material include: sulfonated hydrocarbons and their salts, such as sodium 1,4-bis(2-ethylhexyl) sulfosuccinate, also known as docusate sodium (CROPOL) and sodium lauryl sulfate (SLS); poloxamers, also referred to as polyoxyethylene-polyoxypropylene block copolymers (PLURONICs, LUTROLs); polyoxyethylene alkyl ethers (CREMOPHOR A, BRIJ); polyoxyethylene sorbitan fatty acid esters (polysorbates, TWEEN); short chain glyceryl mono-alkylates (HODAG, IMWITTOR, MYRJ); polyglycolized glycerides (GELUCIREs); mono- and di-alkylate esters of polyols, such as glycerol; nonionic surfactants such as polyoxyethylene 20 sorbitan monooleate, (polysorbate 80, sold under the trademark TWEEN 80, available commercially from ICI); polyoxyethylene 20 sorbitan monolaurate (Polysorbate 20, TWEEN 20); polyeth-ylene (40 or 60) hydrogenated castor oil (available under the trademarks CREMOPHOR® RH40 and RH60 from BASF); polyoxyethylene (35) castor oil (CREMOPHOR® EL).

In some embodiments of the invention the SR formulation may further comprise at least one precipitation inhibitor, wherein the precipitation inhibitors reduce the rate at which the API precipitates from an aqueous solution. Suitable precipitation inhibitors of the invention are chemically inert and in particular do not chemically interact or affect the API. Further, the precipitation inhibitors have to be pharmaceutically acceptable. In addition, the precipitation inhibitors need to be soluble in an aqueous solution under physiological conditions (pH, temperature, osmolarity). The solubility should be at least 0.1 mg/ml over at least a partial pH range between pH 1-8. Precipitation inhibitors may be neutral or ionizable. In the art different polymers are known as widely suitable precipitation inhibitors for different API.

One suitable class of precipitation inhibitor polymers for use with the SR formulations of the present invention comprises neutral non-cellulosic polymers. Exemplary polymers included, but are not limited to: are vinyl polymers and copolymers having substituents of hydroxyl, alkyl acyloxy, or cyclic amido; polyvinyl alcohols that have at least a portion of their repeat units in the unhydrolyzed (vinyl acetate) form; polyvinyl alcohol polyvinyl acetate copolymers; polyvinyl pyrrolidone; polyoxyethylene-polyoxypropylene copolymers, also known as poloxamers; and poly-ethylene polyvinyl alcohol copolymers.

Another suitable class of precipitation inhibitor polymers for use with the SR formulations of the present invention comprises ionizable non-cellulosic polymers. Exemplary polymers included, but are not limited to: carboxylic acid-functionalized vinyl polymers, such as the carboxylic acid functionalized polymethacrylates and carboxylic acid functionalized polyacrylates such as the EUDRAGITS® manufactured by Degussa, of Malden, Mass.; amine-functionalized polyacrylates and polymethacrylates; proteins; and carboxylic acid functionalized starches such as starch glycolate.

Non-cellulosic polymers that are amphiphilic are copolymers of a relatively hydrophilic and a relatively hydrophobic monomer. Examples include acrylate and methacrylate copolymers, and polyoxyethylene-polyoxypropylene copolymers. Exemplary commercial grades of such copolymers include the EUDRAGITS, which are copolymers of methacrylates and acrylates, and the PLURONICS supplied by BASF, which are polyoxyethylene-polyoxypropylene copolymers.

A preferred class of polymers comprises ionizable and neutral cellulosic polymers with at least one ester- and/or ether-linked substituent in which the polymer has a degree of substitution of at least 0.1 for each substituent.

Amphiphilic cellulosics comprise polymers in which the parent cellulosic polymer has a degree of substitution of at least one relatively hydrophobic substituent of at least 0.1. Hydrophobic substituents may be essentially any substituent that, if substituted to a high enough level or degree of substitution, can render the cellulosic polymer essentially aqueous insoluble.

Examples of hydrophobic substituents include ether-linked alkyl groups such as methyl, ethyl, propyl, butyl, etc.; or ester-linked alkyl groups such as acetate, propionate, butyrate, etc.; and ether- and/or ester-linked aryl groups such as phenyl, benzoate, or phenylate. Hydrophilic regions of the polymer can be either those portions that are relatively unsubstituted, since the unsubstituted hydroxyls are themselves relatively hydrophilic, or those regions that are substituted with hydrophilic substituents. Hydrophilic substituents include ether- or ester-linked nonionizable groups such as the hydroxy alkyl substituents such as hydroxyethyl, hydroxypropyl, and the alkyl ether groups such as ethoxy ethoxy or methoxy ethoxy. Particularly preferred hydrophilic substituents are those that are ether- or ester-linked ionizable groups such as carboxylic acids, thiocarboxylic acids, substituted phenoxy groups, amines, phosphates or sulfonates.

One class of cellulosic polymers comprises neutral polymers, meaning that the polymers are substantially non-ionizable in aqueous solution. Such polymers contain non-ionizable substituents, which may be either ether-linked or ester-linked. Exemplary ether-linked non-ionizable substituents include: alkyl groups, such as methyl, ethyl, propyl, butyl, etc.; hydroxy alkyl groups such as hydroxymethyl, hydroxyethyl, hydroxypropyl, etc.; and aryl groups such as phenyl. Exemplary ester-linked non-ionizable substituents include: alkyl groups, such as acetate, propionate, butyrate, etc.; and aryl groups such as phenylate. However, when aryl groups are included, the polymer may need to include a sufficient amount of a hydrophilic substituent so that the polymer has at least some water solubility at any physiologically relevant pH in the range from 1 to 8.

Exemplary non-ionizable polymers that may be used as the polymer include: hydroxypropyl methyl cellulose acetate, hydroxypropyl methyl cellulose, hydroxypropyl cellulose, methyl cellulose, hydroxyethyl methyl cellulose, hydroxyethyl cellulose acetate, and hydroxyethyl ethyl cellulose.

A preferred set of neutral cellulosic polymers are those that are amphiphilic. Exemplary polymers include hydroxypropyl methyl cellulose and hydroxypropyl cellulose acetate, where cellulosic repeat units that have relatively high numbers of methyl or acetate substituents relative to the unsubstituted hydroxyl or hydroxypropyl substituents constitute hydrophobic regions relative to other repeat units on the polymer.

A preferred class of cellulosic polymers comprises polymers that are at least partially ionizable at physiologically relevant pH and include at least one ionizable substituent, which may be either ether-linked or ester-linked. Exemplary ether-linked ionizable substituents include: carboxylic acids, such as acetic acid, propionic acid, benzoic acid, salicylic acid, alkoxy benzoic acids such as ethoxy benzoic acid or propoxy benzoic acid, the various isomers of alkoxy phthalic acid such as ethoxy phthalic acid and ethoxy isophthalic acid, the various isomers of alkoxy nicotinic acid such as ethoxy nicotinic acid, and the various isomers of picolinic acid such as ethoxy picolinic acid, etc.; thiocarboxylic acids, such as thioacetic acid; substituted phenoxy groups, such as hydroxy phenoxy, etc.; amines, such as aminoethoxy, diethyl aminoethoxy, trimethyl aminoethoxy, etc.; phosphates, such as phosphate ethoxy; and sulfonates, such as sulphonate ethoxy. Exemplary ester linked ionizable substituents include: carboxylic acids, such as succinate, citrate, phthalate, terephthalate, isophthalate, trimellitate, and the various isomers of pyridine dicarboxylic acid, etc.; thiocarboxylic acids, such as thiosuccinate; substituted phenoxy groups, such as amino salicylic acid; amines, such as natural or synthetic amino acids, such as alanine or phenylalanine; phosphates, such as acetyl phosphate; and sulfonates, such as acetyl sulfonate. For aromatic-substituted polymers to also have the requisite aqueous solubility, it is also desirable that sufficient hydrophilic groups such as hydroxypropyl or carboxylic acid functional groups be attached to the polymer to render the polymer aqueous soluble at least at pH values where any ionizable groups are ionized. In some cases, the aromatic group may itself be ionizable, such as phthalate or trimellitate substituents.

Exemplary cellulosic polymers that are at least partially ionized at physiologically relevant pHs include: hydroxypropyl methyl cellulose acetate succinate, hydroxypropyl methyl cellulose succinate, hydroxypropyl cellulose acetate succinate, hydroxyethyl methyl cellulose succinate, hydroxyethyl cellulose acetate succinate, hydroxypropyl methyl cellulose phthalate, hydroxyethyl methyl cellulose acetate succinate, hydroxyethyl methyl cellulose acetate phthalate, carboxyethyl cellulose, carboxymethyl cellulose, carboxymethyl ethyl cellulose, cellulose acetate phthalate, methyl cellulose acetate phthalate, ethyl cellulose acetate phthalate, hydroxypropyl cellulose acetate phthalate, hydroxypropyl methyl cellulose acetate phthalate, hydroxypropyl cellulose acetate phthalate succinate, hydroxypropyl methyl cellulose acetate succinate phthalate, hydroxypropyl methyl cellulose succinate phthalate, cellulose propionate phthalate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridine dicarboxylate, salicylic acid cellulose acetate, hydroxypropyl salicylic acid cellulose acetate, ethyl benzoic acid cellulose acetate, hydroxypropyl ethyl benzoic acid cellulose acetate, ethyl phthalic acid cellulose acetate, ethyl nicotinic acid cellulose acetate, and ethyl picolinic acid cellulose acetate.

Exemplary cellulosic polymers that meet the definition of amphiphilic, having hydrophilic and hydrophobic regions include polymers such as cellulose acetate phthalate and cellulose acetate trimellitate where the cellulosic repeat units that have one or more acetate substituents are hydrophobic relative to those that have no acetate substituents or have one or more ionized phthalate or trimellitate substituents.

A particularly desirable subset of cellulosic ionizable polymers are those that possess both a carboxylic acid functional aromatic substituent and an alkylate substituent and thus are amphiphilic. Exemplary polymers include cellulose acetate phthalate, methyl cellulose acetate phthalate, ethyl cellulose acetate phthalate, hydroxypropyl cellulose acetate phthalate, hydroxypropyl methyl cellulose phthalate, hydroxypropyl methyl cellulose acetate phthalate, hydroxypropyl cellulose acetate phthalate succinate, cellulose propionate phthalate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridine dicarboxylate, salicylic acid cellulose acetate, hydroxypropyl salicylic acid cellulose acetate, ethyl benzoic acid cellulose acetate, hydroxypropyl ethyl benzoic acid cellulose acetate, ethyl phthalic acid cellulose acetate, ethyl nicotinic acid cellulose acetate, and ethyl picolinic acid cellulose acetate.

Another particularly desirable subset of cellulosic ionizable polymers are those that possess a non-aromatic carboxylate substituent. Exemplary polymers include hydroxypropyl methyl cellulose acetate succinate, hydroxypropyl methyl cellulose succinate, hydroxypropyl cellulose acetate succinate, hydroxyethyl methyl cellulose acetate succinate, hydroxyethyl methyl cellulose succinate, hydroxyethyl cellulose acetate succinate, and carboxymethyl ethyl cellulose.

While specific polymers have been listed as being suitable for use in the SR formulations of the present invention, blends of such polymers may also be suitable. Thus "polymer" within the present invention includes blends of polymers in addition to a single species of polymer. In particular, it has been found that ionizable cellulosic polymers such as HPMCAS function best over particular pH ranges. For example, HPMCAS aqueous properties are a function of the degree of substitution of each of the substituents: hydroxypropoxy, methoxy, acetate, and succinate, as well as the pH of the use environment. For example, HPMCAS is manufactured by Shin-Etsu, and sold under the trade name AQOAT as three different grades that differ in their levels of substituents and therefore their properties as a function of pH. Thus, it has been found in in vitro tests, that the H grade of HPMCAS is preferred for inhibition of crystallization in a pH 6.5 environment. The H grade of HPMCAS has 22-26 wt% methoxy, 6 10 wt% hydroxypropoxy, 10-14 wt% acetate, and 4-8 wt% succinate groups. At lower pH values, say 5 to 6, the M grade of HPMCAS is preferred. The M grade of HPMCAS has 21-25 wt% methoxy, 5-9 wt% hydroxypropoxy, 7-11 wt% acetate, and 10-14 wt% succinate groups. It has also been found that in an environment where the pH may be variable, such as in the G! tract of a mammal, a mixture of two or more grades may be preferred for optimized solubility, absorption and bioavailability of the API.

Another preferred class of polymers consists of neutralized acidic polymers. By "neutralized acidic polymer" is meant any acidic polymer for which a significant fraction of the "acidic moieties" or "acidic substituents" have been "neutralized"; i.e., exist in their deprotonated form. By "acidic polymer" is meant any polymer that possesses a significant number of acidic moieties. In general, a significant number of acidic moieties would be greater than or equal to about 0.1 milliequivalents of acidic moieties per gram of polymer. "Acidic moieties" include any functional groups that are sufficiently acidic that, in contact with or dissolved in water, can at least partially donate a hydrogen cation to water and thus increase the hydrogen-ion concentration. This definition includes any functional group or "substituent", as it is termed when the functional group is covalently attached to a polymer that has a pKa of less than about 10. Exemplary classes of functional groups that are included in the above description include carboxylic acids, thiocarboxylic acids, phosphates, phenolic groups, and sulfonates. Such functional groups may make up the primary structure of the polymer such as for polyacrylic acid, but more generally are covalently attached to the backbone of the parent polymer and thus are termed "substituents".

The addition of pH modifiers such as acids, bases, or buffers may be beneficial, retarding the dissolution of the API or, alternatively, enhancing the rate of dissolution of API, depending on the API and the respective bases or acids.

Conventional matrix materials, complexing agents, solubilizers, fillers, disintegrating agents (disintegrants), or binders may also comprise up to 90 wt% of the dosage form.

Non-limiting examples of fillers, or diluents include lactose, lactose monohydrate (Neusillin), mannitol, xylitol, microcrystalline cellulose, dibasic calcium phosphate (anhydrous and dihydrate) and starch.

Non-limiting examples of disintegrants include sodium starch glycolate, sodium alginate, carboxy methyl cellulose sodium, methyl cellulose, and croscarmellose sodium, and crosslinked forms of polyvinyl pyrrolidone such as those sold under the trade name CROSPOVIDONE (available from BASF Corporation).

Non-limiting examples of binders include methyl cellulose, microcrystalline cellulose, starch, polyvinyl pyrrolidone (PVP), copovidone and gums such as guar gum, and tragacanth.

Non-limiting examples of lubricants include magnesium stearate, calcium stearate, stearic acid, triglycerides of stearic acid, Aerosil-200, hydrogenated vegetable oils, and palmitic acid.

Non-limiting examples of preservatives include sulfites (an antioxidant), benzalkonium chloride, methyl paraben, propyl paraben, benzyl alcohol and sodium benzoate.

Non-limiting examples of suspending agents or thickeners include xanthan gum, starch, guar gum, sodium alginate, carboxymethyl cellulose, sodium carboxymethyl cellulose, methyl cellulose, hydroxypropyl methyl cellulose, poly-acrylic acid, silica gel, aluminium silicate, magnesium silicate, and titanium dioxide.

Non-limiting examples of anti-caking agents or fillers include silicon oxide and lactose.

Non-limiting examples of solubilizers include ethanol, propylene glycol or polyethylene glycol.

Other conventional excipients may be employed in the SR formulation according to the embodiments of the present invention, including those well-known in the art. Generally, excipients such as pigments, lubricants, flavorants, and so forth may be used for customary purposes and in typical amounts without adversely affecting the properties of the formulations.

Further SR formulations according to the invention may additionally comprise surface coatings. Surface coatings may be employed for aesthetic purposes or for dimensionally stabilizing the compressed dosage form. The surface coating may be carried out using any conventional coating agent which is suitable for oral use. The coating may be carried out using any conventional technique employing conventional ingredients. A surface coating may, for example, be obtained using a quick dissolving film using conventional polymers, such as hydroxypropyl methyl cellulose, hydroxypropyl cellulose, carboxymethyl cellulose, polyvinyl alcohol poly methacrylates, or other well-known coatings in the art.

## Determination of solubility enhancement and characterization of sustained release profiles of SR formulations containing solubility enhanced API

Several methods, such as an in vitro dissolution test or a membrane permeation test may be used to determine and quantify the enhanced solubility of the solubility enhanced API formulations and the SR of the invention. An in vitro dissolution test may be performed by adding the solubility-enhanced API or SR formulation to a dissolution test media, such as model fasted duodenal (MFD) solution, phosphate buffered saline (PBS) solution, simulated intestinal or gastric buffer solution, or water and agitating to promote dissolution. Typically, standard in-vitro dissolution tests are performed in the following aqueous medium: 0.1 N or 0.01 N HCl or buffer adjusted to pH 6.8. An appropriate PBS solution is an aqueous solution comprising 20 mM $Na_2HPO_4$, 47 mM $KH_2PO_4$, 87 mM NaCl, and 0.2 mM KCl, adjusted to pH 6.5 with NaOH. An appropriate MFD solution is the same PBS solution with an additional 7.3 mM sodium taurocholic acid and 1.4 mM of 1-palmitoyl-2-oleyl-sn-glycero-3-phosphocholine. Appropriate simulated intestinal buffer solutions include (1) 50 mM $NaH_2PO_4$ and 2 wt% sodium lauryl sulfate, adjusted to pH 7.5, (2) 50 mM $NaH_2PO_4$ and 2 wt% sodium lauryl sulfate, adjusted to pH 6.5, and (3) 6 mM $NaH_2PO_4$, 150 mM NaCl, and 2 wt% sodium lauryl sulfate, adjusted to pH 6.5. Water is a preferred dissolution media for some fast-precipitating salts.

In one method for evaluating whether the SR formulation includes a solubility-enhanced API form, the solubility-enhanced form of the API when tested in an in vitro dissolution test meets at least one, and preferably both, of the following conditions. The first condition is that the solubility-enhanced API provides a higher maximum dissolved drug concentration (MDC) of the API in the in vitro dissolution test relative to pure API or a control composition consisting of the non-solubility enhanced form of the API. The second condition means, once the SR formulation of the solubility enhanced API is added into the test environment, the solubility-enhanced API provides a higher aqueous concentration of dissolved API relative to the control composition containing NON solubility enhanced API or the pure API.

Performance of Solubility Enhancement of API

For step one an in vitro dissolution test may be performed by adding the solubility-enhanced API using dissolution test as defined in US Pharmacopeia standard (chapter <711> "Dissolution") with any of their proposed apparatus and conditions; e.g., 37 °C and paddle rotation of 50 rpm, with either water or standard aqueous media at pH 1, pH 2, water or buffer pH 6.8 according to US Pharmacopeia standard (chapter <711> "Dissolution"). A different aqueous media may be selected if required for stability reasons of the API; e.g., decompensation of the at pH 6,8 or change of the polymorph of the API, e.g. salt formation of a base under low pH conditions and vis versa base formation of an API salt with a strong acid at higher pH values. Preferably aqueous conditions are selected to maintain an unchanged API polymorph or the most relevant conditions at application site, e.g. SR formation are tested at pH 6.8 because the main portion of the API is absorbed under these conditions. The amount dissolved is determined either by measuring the concentration at the defined time points and the amount dissolved is plotted versus time. The amount at each time point is determined by a suitable assay method, e.g., UV for API with sufficient extinction coefficient or HPLC with low extinction coefficient. The released amount result is calculated as % of the tested dose of the API over a period of 180 minutes. Dissolution of the solubility enhanced API is compared with the dissolution of the pure API or a formulation containing the NON solubility enhanced API in the same amount of dissolution media. The first condition is that the solubility-enhanced API provides a higher or faster reached maximum amount of dissolve/released drug (MDR) in at least at one time point measured in vitro dissolution test compared to a control composition consisting of the non-solubility enhanced form of the API. Preferably, the solubility-enhanced API provides an MDR of the API in aqueous solution that is at least 200% that of the control composition (non -enhanced API), more preferably at least 300%, and most preferably at least 400%.

Performance of SR formulation of solubility enhanced API

In a second step SR formulation of solubility enhanced API meeting the above criteria are prepared and the dissolution of the SR formulation is determined using the same method as described for testing the performance of solubility enhanced API. The pH of the dissolution media may be altered and additives to achieve "sink conditions" may be added. The dissolution test is performed over a period of at least 180 minutes, at least 240 minutes, at least 300 minutes, at least 360 minutes. SR Formulation according to this invention are (a) maintaining solubility enhancement of the API and (b) are showing SR properties.

Solubility enhancement of API is maintained if the MDR is equal to at least 25%, at least 30%, at least 40%, at least 50% of the achieved solubility enhancement of step one at the maximum of the dissolution test of the SR formulation but not before 120 minutes. Preferred the solubility enhancement determination is performed in a head-to-head experiment meaning that a SR formulation containing the solubility enhanced API is compared with a comparable SR formulation containing the pure API. However, this may be negligible if the MDR of SR formulation containing the solubility enhanced API reaches far more than 25%, preferably more than 30%, preferably more than 40% or preferably more than 50% of the solubility result of the pure API in step 1.

Sustained release dissolution profile can follow a first order, a zero order or an ascending characteristic. All of them are significant different from an Immediate release profile, e.g., solubility enhanced API. Common for all SR dissolution profiles is (a) MDR at 30 minutes, at 60 minutes is less than 70% of the MDR at the end of the tested range and (b) the slope at any of 2, of 3, of 4 intervals of maximum 30 minutes within the tested range is more than 1.

The given definition for SR release formulation is supported by many examples comparing IR and SR formulation and is widely accepted by those skilled in art.

Some embodies of this invention may be formulated as combination of IR release and SR release formulation containing the solubility enhanced API. For those formulation the performance indicating parameters need to be adopted to the SR portion only.

Analogously to the above evaluations of the solubility-enhanced API or the SR formulations of the invention, these may also be tested and evaluated in the presence of precipitation inhibitors. Several methods, such as an in vitro dissolution test or a membrane permeation test may be used to evaluate precipitation inhibitors and the degree of concentration enhancement provided by the precipitation inhibitors. An in vitro dissolution test may be performed by adding the solubility-enhanced API or the SR formulation of the invention together with the precipitation inhibitor to MFD or PBS or simulated intestinal buffer solution and agitating to promote dissolution. To evaluate the utility of precipitation inhibitors in use environments at other pH values, it may be desirable to use other similar dissolution media that have pH values adjusted to other values. For example, an acid such as HCl or $H_3PO_4$ may be added to PBS or MFD to adjust the pH of the solution to 6.0 or 5.0 and then used in the following dissolution tests. A solubility-enhanced API or SR formulation of the invention together with the precipitation inhibitor, when tested in an in vitro dissolution test meets at least one, and preferably both, of the following conditions. The first condition is that the solubility-enhanced API or SR formulation and the precipitation inhibitor provide a higher maximum dissolved drug concentration (MDC) of API in the in vitro dissolution test relative to a control composition. The control composition consists of the solubility-enhanced API or the SR formulation alone (without the precipitation inhibitor). That is, once the solubility-enhanced API or SR formulation and the precipitation inhibitor are introduced into a use environment, the solubility-enhanced API or SR formulation and precipitation inhibitor provide a higher aqueous concentration of dissolved API relative to the control composition. It is important to note that the solubility-enhanced API or SR formulation and precipitation inhibitor are dissolution tested independently of the dosage form so that the sustained release means do not interfere with evaluation of the degree of solubility improvement. Preferably, the solubility-enhanced API or SR formulation and precipitation inhibitor provide an MDC of API in aqueous solution that is at least 125% that of the control composition, more preferably at least 150%, at least 175%, at least 200%, and most preferably at least 300%.

Alternatively, an in vitro membrane-permeation test may be used to evaluate the precipitation inhibitor. In this test, as described above, the solubility-enhanced API or SR formulation and the precipitation inhibitor are placed in, dissolved in, suspended in, or otherwise delivered to the aqueous solution to form a feed solution. A typical in vitro membrane-permeation test to evaluate precipitation inhibitors can be conducted by (1) administering a sufficient quantity of test composition (that is, the solubility-enhanced API or SR formulation ziprasidone and precipitation inhibitor) to a feed solution, such that if all of the API is dissolved, the theoretical concentration of API would exceed the equilibrium concentration of the API by a factor of at least 3; (2) in a separate test, adding an equivalent amount of control composition to an equivalent amount of test medium; and (3) determining whether the measured maximum flux of API provided by the test composition is at least 1.25-fold that provided by the control composition. The solubility-enhanced API or SR formulation and precipitation inhibitor, when dosed to an aqueous use environment, provide a maximum flux of API in the above test that is at least about 125% the maximum flux provided by the control composition. Preferably, the maximum flux provided by the test composition is at least about 150%, preferably at least about 175%, more preferably at least about 200%, and even more preferably at least about 300% that provided by the control composition.

## Processes for preparation of compositions and formulations of the invention

Another aspect of the present invention are methods and processes of preparing both the API-inclusion complexes for enhanced solubility API preparations and/or methods and processes for preparing the SR formulations including the solubility-enhanced API-inclusion complexes.

Preparation of solubility-enhanced API inclusion complexes

In one embodiment of the methods of the invention preparing the API-inclusion complex involves admixing the API or a pharmaceutically acceptable salt thereof with a suitable selected complex forming molecule such as α-, β-, or γ-cyclodextrin or its derivates in a molar ratio of the compounds as provided above. In a preferred embodiment the API and complex forming agent are provided in a molar ratio of 1:1 or 1:2 under stirring at a temperature from about room temperature to boiling temperature of the reaction mixture in an aqueous or ethanolic medium, and subsequently cooling the reaction mixture to about 0° to 5° C, and possibly isolating the desired complex if required. In a preferred embodiment isolation or purification of the solubility enhanced API is not required.

The admissible deviation from the 1: 1 or 1:2, respectively molar ratio is ±20% at the most, i.e., the molar ratio can be 1:0.8 to 1.2 or 1:1.6 to 2.4.

In a preferred embodiment of the invention the invention relates to preparation of an API:CD inclusion complex, although other complex-forming agents besides CD may be used.

The API:CD inclusion complex can be prepared using five different methodologies, namely kneading (KND), solid dispersion (SDP), spray drying (SD), freeze-drying (FDY), and Fluid Bed Coating (FBC) which are described below. In a preferred embodiment the mixing ratio of API to CD is 1:1 or 1:2 molar ratio. The resulting powder of the API:CD inclusion complex can subsequently undergo physicochemical characterization and usually should be stored at 4°C or at room temperature if stabilized with a suitable carrier.

### Kneading method (KND)

In order to prepare the complex using the kneading method, the CD and API are transferred to a glass mortar, mixed and homogenized with water in the proportion of 1:4 (solid-water) to produce the slurry, which is kneaded for an appropriate amount of time typically about 45 min to 1 h. Following this period, the material is dried at 40 °C for about 24 h.

### Solid dispersion (SDP)

Complexes may also be prepared by the solid dispersion method. Depending on the CD-Derivative initially the CD is dissolved in water at concentration of about 1- 40 w/w%. The API is dissolved in a suitable solvent such as, e.g., ethyl alcohol or another water-miscible solvent, and the solution is subsequently added to the CD solution in order to allow complexation. This suspension obtained is kept in an ultrasonic bath for about 10 min or stirred for 16 to 24 hours, and afterwards the solvent is removed while stirring by vacuum drying at room temperature. The resulting solids are milled.

### Spray-drying (SD)

Complexes may also be prepared by Spray drying method. Depending on the CD derivative initially the CD is dissolved in water at concentration of about 1- 40 w/v%. The API is dissolved in a suitable solvent such as, e.g., ethyl alcohol or another water-miscible solvent, and the solution is subsequently added to the CD solution in order to allow complexation. This suspension obtained is kept in an ultrasonic bath for about 10 min or stirred for 16 to 24 hours, filtered and dried in a spray dryer. Advantage of this method is immediate subsequent usability of the obtained API-CD complex.

*Freeze-drying (FDY)*

The API is dispersed in water or depending on the solubility of the API dissolved in hydrochloric acid or potassium or sodium hydroxide or a suitable water miscible organic solvent. The API solution is mixed with a solution containing CD and stirred at room temperature for about 24 h. The pH is adjusted to about 4.0 to 10.0 with depending on initial pH either 0.1 mol/l potassium or sodium hydroxide or 0.1 mol/l hydrochloric acid or alternative suitable bases or acids maintaining a clear solution. Then, the solution is freeze-dried and the powder obtained contains the solubility enhanced API.

*Fluid Bed Coating (FBC)*

Preferred method of this invention is preparation of the complexes by the fluid bed coating method. Initially CD (e.g.HP-β-cyclodextrin) is dissolved in water at concentration of about 30 w/w%. The API is dissolved in a suitable solvent such as, e.g., ethyl alcohol or another water-miscible solvent, and the solution is subsequently added to the CD solution in order to allow complexation. This suspension obtained is kept stirring for 16-24 hours and coated on sugar or cellulose beads. The advantage of this method is the immediate usage in the preferred SR formulation technology.

Preparation of SR formulations

The SR formulations of the present invention comprising solubility-enhanced API and precipitation inhibitor may be prepared by dry- or wet mixing the API or API mixture with a carrier and/or the precipitation inhibitor together with excipients as described above to from a matrix SR release composition. Mixing processes include physical processing, including intimate physical mixture, direct compression as well as wet-granulation, extrusion/melt granulation, dry granulation and coating processes.

For example, mixing methods include convective mixing, shear mixing, or diffusive mixing. Convective mixing involves moving a relatively large mass of material from one part of a powder bed to another, by means of blades or paddles, revolving screw, or an inversion of the powder bed. Shear mixing occurs when slip planes are formed in the material to be mixed. Diffusive mixing involves an exchange of position by single particles. These mixing processes can be performed using equipment in batch or continuous mode. Tumbling mixers (e.g., twin-shell) are commonly used equipment for batch processing. Continuous mixing can be used to improve composition uniformity.

The components of the compositions of this invention may also be combined by dry- or wet-granulating processes using any conventional pharmaceutical manufacturing process.

In another embodiment of the invention SR polymer layers of the formulation with or without the precipitation inhibitor may be coated around the solubility-enhanced API using any conventional method. A method is a spray drying process. The term spray-drying is used conventionally and broadly refers to processes involving breaking up liquid mixtures or suspensions into small droplets (atomization) and rapidly removing solvent from the droplets in a container where there is a strong driving force for evaporation of solvent. Fluid bed coating, in this respect as a special type of spray drying is the most preferred method for preparing SR formulations containing solubility enhanced APIs, In the same type of fluid bed equipment used in a first step to solidify the solution of the API-CD complex by coating application on sugar or cellulose pellets the SR polymer solution is subsequently applied as a second step on top of the solubility enhanced API layer.

Other suitable carrier materials commonly known in the field may be used in the same way to prepare SR formulations. Carrier materials may be selected from, but are not limited to, sugars and sugar derivates, HPMC, PEO, tartaric acid and PVP and other pharmaceutically acceptable carriers.

The solvent is chosen based on the following characteristics: (1) the polymer is soluble in the solvent; and (2) the solvent is relatively volatile. Preferred solvents include alcohols such as methanol, ethanol, n-propanol, iso-propanol, and butanol; ketones such as acetone, methyl ethyl ketone and methyl iso-butyl ketone; esters such as ethyl acetate and propyl acetate; and various other solvents such as acetonitrile, methylene chloride, toluene, THF, cyclic ethers, and 1,1,1-trichloroethane. A preferred solvent is ethanol and 2-Propanol. Mixtures of solvents may also be used, e.g., mixtures with water can be used as long as the polymer is sufficiently soluble to make the spray-drying process practicable. In some cases, it may be desired to add a small amount of water to aid solubility of the hydrophilic pore forming polymer or other water-soluble excipients in the SR release polymer spray solution.

To coat the solubility-enhanced API by spray drying, first a suspension of solubility enhanced API and dissolved polymer is formed in a solvent. The relative amounts of solubility enhanced API suspended in the solvent and polymer dissolved in the solvent are chosen to yield the desired API to polymer ratio in the resulting particles. For example, if a particle having an API to polymer ratio of 0.33 (25 wt% API) is desired, then the spray solution comprises 1-part crystalline solubility enhanced API particles and 3 parts polymers dissolved in the solvent. The total solids content of the spray solution is preferably sufficiently high so that the spray solution results in efficient production of the particles. The total solids content refers to the amount of solid solubility-enhanced API, dissolved polymer and other excipients dissolved in the solvent. For example, to form a spray solution having a 5 wt% dissolved solids content and which results in a particle having a 25 wt% API loading, the spray solution would comprise 1.25 wt% API, 3.75 wt% polymer and 95 wt% solvent. To achieve good yield, the spray solution preferably has a solids content between 3 wt% and 10%. However, the dissolved solids content should not be too high, or else the spray solution may be too viscous to atomize efficiently into small droplets.

Often it is desirable for the particle size of the solubility-enhanced API to be relatively small. This promotes satisfactory coating of the API particles by the polymer. Thus, it is generally preferred for the API particles to have a volume average diameter of less than about 10 $\mu$m and preferably less than about 5 $\mu$m.

In some embodiments of the invention sodium alginate is used for the preparation of the matrix, which is present in pharmaceutical preparations in an amount from 40 to 70% by weight, from 40 to 60%, from 40 to 50% by weight. In alternative embodiments of the invention high-molecular celluloses, such as methyl cellulose, hydroxypropyl cellulose or hydroxypropyl methylcellulose of various viscosity grades, such as hydroxypropyl methylcellulose having a viscosity of 4000 cp are used for the preparation of the matrix, which is present in pharmaceutical preparations in an amount from 10 to 50% by weight, from 10 to 40% by weight, from 10 to 30% by weight, from 10 to 25% by weight, from, from 20 to 30% by weight, from 30 to 50% by weight, from 30 to 0% by weight.

**Determination of entrapment efficiency**

The SR formulations of the invention can be analysed for their critical physicochemical properties to provide a characterization of the particles and for further optimization of properties and processes of preparing the SR formulation.

Highly relevant for the assessment of the solubility-enhances API and the processes of preparation is to determine the entrapment efficiency for the API within the API inclusion complexes. Entrapment efficiency (EE) of the API can be determined indirectly using ultrafiltration technique. API-loaded solution of inclusion complexes is placed in the upper chamber of a membrane concentrator and centrifuged for about 15 min at about 4000 rpm depending on the device operational requirements. Such the entrapped and unentrapped API are separated by ultrafiltration. The unentrapped API in the lower chamber of the filtrate is detected and quantified by HPLC using UV/VIS spectroscopy. The total API concentration is determined as follows: API loaded dispersions are dissolved in an appropriate solvent, e.g., methanol and chloroform (1:1), and treated in an ultrasonic in water bath for about 10 min at about 60°C to disrupt the particles. The obtained suspension is also filtered membrane filters, and the total API content can be determined following the same procedure as above. This results in the following calculation for API entrapment:

$$EE\% = \frac{W_T - W_F}{W_T} \times 100\%$$

where the $W_T$ is the amount of the initial API (total API) and the $W_F$ is the amount of unentrapped API in the filtrate. Instead of ultrafiltration it is also possible to use ultracentrifugation to separate the unentrapped API from the API complexes.

A much easier and practical alternative way to determine the entrapment efficiency suitable to be used as an in-process control during manufacturing of SR embodiments according in this invention is herewith disclosed. The Solubility enhanced API-cyclodextrin complex formed in the first step of this invention as well and the SR formulation of the said solubility enhanced API prepared in the second step of this invention by the methods described above are tested for solubility respectively sustained release characteristic in well-established dissolution testing apparatus described in US Pharmacopeia standard (chapter <711> "Dissolution") in aqueous media under conditions relevant for the in-vivo and in-vitro environment of use. As explained above the pure API is less soluble in the amount of the test media. Thus, efficient entrapment is proven if the dissolved solubility enhanced API-complex respectively the API-complex released from the SR formulation does not show a decrease of API-content by more than 20%, most preferably more than 10%, of the MDR.

In some cases, it may be necessary to add cyclodextrin to the dissolution media to maintain dissolution "sink conditions" to ensure that the API released from the SR formulation remains accessible to analytical assay methods and is not hidden because of an artificial shift of the post release solubility equilibrium from inside to outside the SR formulation.

**EXAMPLES**

As proof-of-concept experiments the following tests were performed by the inventors.

**Experiment 1: Production of Solubility enhanced Axitinib Pellets with MCC Pellets 700 starter pellets**

Axitinib is soluble at pH < 1,5 and very low soluble at higher pH values. Solubility trials have been performed to determine optimal complexation conditions and molecular ratio between Axitinib and Hydroxypropyl-$\beta$-cyclodextrin. Based on these results the following experimental design was selected.

1.0 g Axitinib was dissolved in a solution of 10.0 g Hydroxypropyl-$\beta$-Cyclodextrin and 5.0 g Hydroxypropyl methylcellulose (HPMC 603) in 72 g HCl-solution pH 1.2 and stirred for 6 hours. The pH of the solution was gradually adjusted to pH 4.0. The obtained mixture A contained the formed solubility enhanced Axitinib-Cyclodextrin inclusion complex.

84.4 g Vivapur® MCC Pellets 700 were loaded into a fluid bed processor and the mixture A. was sprayed onto the MCC Pellets and dried to result in solubility enhanced Axitinib:CD immediate release (IR) pellets (Pellets A).

**Experiment 2: Production of NON-Solubility enhanced Axitinib Pellets with MCC Pellets 700 starter pellets**

As a comparative negative control experiment to experiment 1 a mixture of 1.0 g Axitinib suspended 20 g Ethanol was added to a solution of 4.0 g Hydroxypropyl methylcellulose (HPMC 603) in 40 g water and kept stirring. The obtained mixture B contains Axitinib without solubility enhancement by a cyclodextrin.

80.0 g Vivapur® MCC Pellets 700 were loaded into a fluid bed processor and the mixture B. was sprayed onto the MCC Pellets and dried to result in NON solubility enhanced Axitinib pellets (Pellets B).

**Experiment 3: Dissolution comparison of solubility enhanced with NON-solubility enhanced Axitinib Pellets**

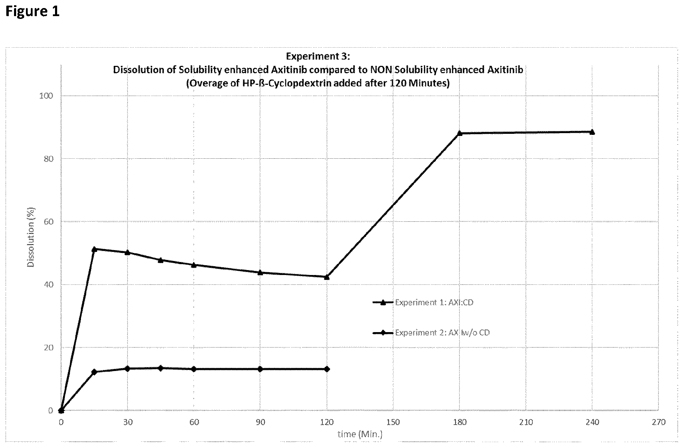

Figure 1 shows the results of in-vitro dissolution experiments wherein the obtained pellets of experiments 1 and 2, respectively are tested in comparison to each other in dissolution apparatus according to US Pharmacopeia standard (chapter <711> "Dissolution") Paddle at 50 rpm in buffer pH 6.8. The results of Fig. 1 clearly show that Axitinib dissolved of the pellets of experiment 1 releases significantly larger amounts (about 4-fold or 5.78 $\mu$g/ml) of Axitinib compared to Axitinib dissolved of the pellets obtained in the control experiment 2 containing NON solubility enhanced Axitinib (1.39 $\mu$g/ml) after 15 Minutes.

After adding an overage of Hydroxypropyl-$\beta$-Cyclodextrin to the dissolution media in order to obtain dissolution "sink" condition the the cumulative amount of Axitinib dissolved increases immediately to 9.2 $\mu$g/ml. This can be explained that a higher dose got dissolved from the pellets but segregated in the filter of the apparatus and/or the Axitinib:CD complex dissociated because of the altered complexation balance in the large amount of dissolution media.

The results are plotted in figure 1

**Experiment 4: Production of Sustained Release Pellets containing solubility enhanced Axitinib:CD complex with MCC 700 starter pellets**

Solubility enhanced AXITINIB pellets of experiment 1 were coated in a second step with a Diffusion Membrane coating as an exemplary sustained release formulation according to the invention.

5.76 g Hydroxypropyl cellulose (pore builder) and 1.28 g propylenglycol (plasticizer) were dissolved in 46 g water and added to a solution of 5.76 g Ethocel® 45 (membrane forming water insoluble polymer) in 115.2 g Ethanol 96% and kept stirring to form an opak solution (mixture A).

80 g Axitinib:CD complex pellets of experiment 1 were loaded into a fluid bed processor and the 80% of mixture A corresponding to an amount of 16% weight gain was sprayed onto the pellets and dried to result in Axitinib:CD SR pellets 16% (Pellets C).

Pellets C did display a weak sustained release effect only. Thus, Pellets C were coated with an additional Diffusion Membrane Coating. For this 160 g Hydroxypropyl cellulose (pore builder) and 1.20 g propylenglycol (plasticizer) were dissolved in 31.2 g water and added to a solution of 5.20 g Ethocel® 45 (membrane forming water insoluble polymer) in 104.0 g Ethanol 96% and kept stirring to form opak solution (mixture B)

80 g Axitinib:CD SR Pellets 16% (Pellets C) were loaded into a fluid bed processor and mixture B was sprayed onto the pellets and dried. After 50% of applying mixture B corresponding to 5% weight gain and 100% respectively corresponding to 10% weight gain samples have been withdrawn labelled as Axitinib:CD SR pellets 5% (Pellets D) and Axitinib:CD pellets 10% (Pellets E) respectively.

**Experiment 5: Dissolution Testing of Diffusion Membrane Sustained Release Pellets containing solubility enhanced Axitinib: CD complex of experiment 4.**

Sustained release Pellets C, D and A containing solubility enhanced Axitinib obtained from experiments 4 were tested in comparison to each other in dissolution apparatus according to US Pharmacopeia standard (chapter <711> "Dissolution") Paddle at 50 rpm in buffer pH 6.8 500 ml. 8 g of HP-$\beta$-Cyclodextrin were added to each dissolution vessel to establish dissolution "sink"-conditions addressing low solubility of Axitinib at pH 6.8 after released from the sustained release pellets.

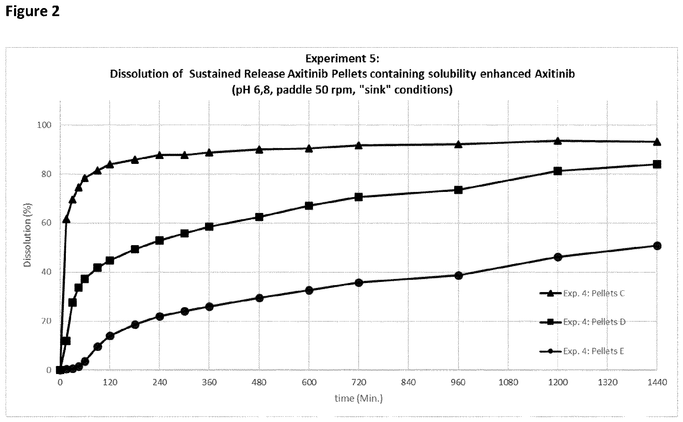

The results plotted in Figures 2 clearly show, that Axitinib is dissolved from the pellets of experiment 4 in a sustained release characteristic defined. Increasing levels of sustained release coating, 16%, 16+5 % and 16+10% reduces the slope of the cumulative release profile and the release rates per time interval. Both are typical properties of a Diffusion Membrane SR technology.

**Claims**

1. A solid pharmaceutical composition for oral administration comprising Axitinib or other kinase inhibitors (API) in a non-covalent inclusion complex with a hydrophilic complex-forming agent, wherein the API-inclusion complex is applied to a pharmaceutical carrier and wherein the pharmaceutical carrier carrying the API-inclusion complex are formulated as a sustained release formulation.

2. The pharmaceutical composition of claim 1 wherein API-inclusion complex is first applied to a pharmaceutical carrier and wherein the pharmaceutical carrier carrying the API-inclusion complex are subsequently formulated as a sustained release formulation.

3. The pharmaceutical composition of any one of the preceding claims wherein the API has low solubility in an aqueous solution of between 5 to 10 wt%, between 3 to 5 wt%, between 1 to 3 wt%, between 0.1 to 1 wt% and between 0.01 to 0.1 wt%, and between 0.1 to 3 wt%, between 0.1 to 5 wt%, between 0.1 to 10 wt% and between 1 to 5 wt%, between 1 to 10 wt% and 3 to 10 wt%, measured in water or another appropriate aqueous media between 4 and 37 °C at buffer pH between 0.05 and 12, preferably between 4 and 37 °C at buffer pH 6.8, more preferably at 20°C at buffer pH 6.8.

4. The pharmaceutical composition of any one of the preceding claims wherein formation of the API-inclusion complex enhances solubility of the API, the solubility-enhanced API provides a maximum dissolved drug concentration of the API in aqueous solution that is at least 125% of the pure API more preferably at least 150%, more preferably at least 175%, more preferably at least 200%, and most preferably at least 300%.

5. The pharmaceutical composition of any one of the preceding claims wherein the pharmaceutical carrier is selected from the group comprising, microcrystalline cellulose and Cellulose derivatives, sugars and sugar derivates, HPMC, PEO, tartaric acid and PVP.

6. The pharmaceutical composition of any one of the preceding claims wherein the complex-forming agent is selected form the group comprising $\alpha$-cyclodextrin, $\beta$-cyclodextrin and $\gamma$-cyclodextrin, ether-derivates of $\alpha$-cyclodextrin, $\beta$-cyclodextrin and $\gamma$-cyclodextrin, acetylated forms of cyclodextrin, hydrophilic cyclodextrins and any mixture thereof.

7. The pharmaceutical composition of any one of the preceding claims wherein API:complex-forming agent molar ratios is between 1:0.5 and 1:5.

8. The pharmaceutical composition of any one of the preceding claims wherein the sustained release formulation is selected from the group comprising matrix sustained release formulation, osmotic sustained release formulations, diffusion membrane formulations, multi-particulate sustained release formulations and multi-layered sustained release formulations.

9. The pharmaceutical composition of claim 8 wherein the dissolution profile of the pharmaceutical composition refers to the release of the solubility-enhanced API and the release profile of sustained release formulation achieves a maximum in-vitro release (MDR) in aqueous media of maximum 70% of the cumulative finally released solubility-enhanced API within 1 h and a release rate of greater zero in more than 2 consecutive time periods of 30 minutes as determined by in-vitro dissolution testing in aqueous media.

10. A process for preparing the pharmaceutical composition according to any one of the preceding claims comprising the steps:

  (i) preparation of an API-inclusion complex comprising API and a complex-forming agent, and
  (ii) preparation of a sustained release formulation comprising the API-inclusion complex of step (i).

11. The process according to claim 10, wherein the API-inclusion complex is prepared by a technique selected from the group comprising kneading, solid dispersion, spray drying, freeze-drying and fluid bed coating.

12. The process according to claim 10, wherein the sustained release formulation is prepared by a technique selected from the group comprising physical mixture, direct compression as well as wet-granulation, extrusion/melt granulation, dry granulation, fluid bed coating and drum coating.

**Figure 1**

**Figure 2**

Experiment 5:
Dissolution of Sustained Release Axitinib Pellets containing solubility enhanced Axitinib
(pH 6,8, paddle 50 rpm, "sink" conditions)

Europäisches
Patentamt

European
Patent Office

Office européen
des brevets

## EUROPEAN SEARCH REPORT

**Application Number**

EP 24 20 6603

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/000756 A1 (SHI SHUIPING [CN] ET AL) 3 January 2019 (2019-01-03) * paragraphs [0002], [0005], [0009], [0287], [0288] * ----- | 1-12 | INV. A61K9/16 A61K9/20 A61K47/50 |
| A | CN 110 051 859 A (LUNAN PHARMACEUTICAL GROUP CORP) 26 July 2019 (2019-07-26) * the whole document * ----- | 1-12 | |

**TECHNICAL FIELDS SEARCHED (IPC)**

A61K

The present search report has been drawn up for all claims

| Place of search | Date of completion of the search | Examiner |
|---|---|---|
| The Hague | 27 March 2025 | Weiss, Marie-France |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6603

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2025

| Patent document cited in search report | | Publication date | Patent family member(s) | | Publication date |
|---|---|---|---|---|---|
| US 2019000756 A1 | | 03-01-2019 | CN | 106902358 A | 30-06-2017 |
| | | | EP | 3395346 A1 | 31-10-2018 |
| | | | JP | 6654702 B2 | 26-02-2020 |
| | | | JP | 2019504042 A | 14-02-2019 |
| | | | US | 2019000756 A1 | 03-01-2019 |
| | | | WO | 2017107895 A1 | 29-06-2017 |
| CN 110051859 A | | 26-07-2019 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180250262 A

**Non-patent literature cited in the description**

- *J. Pharm. Sci.*, 1975, vol. 64, 1585